# EUROPEAN PATENT SPECIFICATION

(11) **EP 2 893 061 B1**
(45) Date of publication and mention of the grant of the patent: **22.05.2019**
(21) Application number: 13834985.7
(22) Date of filing: 05.09.2013
(51) Int. Cl.: C40B 30/04

(54) **METHODS FOR DISCOVERING THERAPEUTIC TARGETS**
VERFAHREN ZUR ENTDECKUNG THERAPEUTISCHER ZIELMOLEKÜLE
PROCÉDÉS DE DÉCOUVERTE DE CIBLES THÉRAPEUTIQUES

(30) Priority: 05.09.2012 US 201261696873 P; 29.08.2013 US 201314014168
(43) Date of publication of application: 15.07.2015
(73) Proprietor: Arizona Board of Regents, a Body Corporate of the State of Arizona acting for and on behalf of Arizona State University, Scottsdale, AZ 85257-9908 (US)
(72) Inventor: JOHNSTON, Stephen, Albert, Tempe, AZ 85284 (US); LEGUTKI, Joseph, Barten, Scottsdale, AZ 85257-9908 (US); KUKREJA, Muskan, Oceanside, CA 92058 (US)
(74) Representative: Avidity IP
(86) International application number: PCT/US2013/058325
(87) International publication number: WO 2014/039718

(56) References cited:
- WO-A2-2008/151146
- US-A1- 2009 176 664
- US-A1- 2011 143 953
- US-A1- 2012 190 574
- R. F. HALPERIN ET AL: "Exploring Antibody Recognition of Sequence Space through Random-Sequence Peptide Microarrays", MOLECULAR & CELLULAR PROTEOMICS, vol. 10, no. 3, 1 March 2011 (2011-03-01), XP055261904, ISSN: 1535-9476, DOI: 10.1074/mcp.M110.000786
- CHEN GUOAN ET AL: "Autoantibody profiles reveal ubiquilin 1 as a humoral immune response target in lung adenocarcinoma", CANCER RESEARCH, AMERICAN ASSOCIATION FOR CANCER RESEARCH, US, vol. 67, no. 7, 1 April 2007 (2007-04-01), pages 3461-3467, XP002613601, ISSN: 0008-5472, DOI: 10.1158/0008-5472.CAN-06-4475
- MICHAEL HECKER ET AL: "Computational analysis of high-density peptide microarray data with application from systemic sclerosis to multiple sclerosis", AUTOIMMUNITY REVIEWS, vol. 11, no. 3, 1 January 2012 (2012-01-01), pages 180-190, XP028445112, ISSN: 1568-9972, DOI: 10.1016/J.AUTREV.2011.05.010 [retrieved on 2011-05-18]

## Description

### BACKGROUND

High-throughput technologies such as DNA, RNA, protein, antibody, and peptide microarrays are often used to examine differences across drug treatments, diseases, transgenic animals, and others. For example, US 2012/0190574 discloses compound arrays for use in profiling samples. The interpretation of the data that is acquired from such high-throughput technologies is not a trivial process: specialized algorithms need to be created, developed, and optimized for analysis. Moreover, limitations of current technologies prevent their application to target identifying screening.

### SUMMARY OF THE INVENTION

The present invention comprises a method of screening for therapeutic targets for an autoimmune disorder comprising:
a) contacting a first peptide array with a first biological sample from an individual with an autoimmune disorder;
b) detecting binding of at least one antibody in the first biological sample with the first peptide array to obtain a first immunosignature profile;
c) contacting a second peptide array with a second biological sample, consisting of a control sample derived from an individual without the autoimmune disorder;
d) detecting binding of at least one antibody in the control sample with the second peptide array to obtain a second immunosignature profile;
e) comparing the first immunosignature profile to the second immunosignature profile and identifying peptide(s) that are associated with the at least one antibody downregulated in the first biological immunosignature profile; and
f) identifying proteins that correspond to the identified peptides as therapeutic targets for the autoimmune disorder;
wherein the autoimmune disorder is selected from the group consisting of Type 1 diabetes, rheumatoid arthritis, multiple sclerosis, inflammatory bowel disease, systemic lupus erythematosus, psoriasis and scleroderma.

### BRIEF DESCRIPTION OF THE FIGURES

**FIGURE 1** is a pathway showing how a self protein/antigen can lead to up-regulation and down-regulation of immunosignature in random sequence peptide microarrays.
**FIGURE 2** is a box plot showing the intensity of 13 peptides by 2 groups of Type I diabetes patients as possessing either high and low GAD-65 RIP titers.
**FIGURE 3** is a linear regression analysis of the relationship between peptide intensity on the immunosignature array and the anti-GAD titers.
**FIGURE 4** Identification of KSFHGRVIQDVVGEPYGGSC (SEQ ID NO. 1) as a peptide of interest. Panel A is a graph showing the relationship of the peptides between the bioinformatic averages of the normalized intensities for wildtype vs the transgenic mice. Panel B is a line graph where the normalized intensities for individual mice are plotted as a line graph. The lines in Panel B represent each individual peptide.
**FIGURE 5** corresponds to BLAST alignments of peptide KSFHGRVIQDVVGEPYGGSC (SEQ ID NO. 1) to the top two mouse proteome candidates. Panel A corresponds to a BLAST alignment of a top likely match: p190RhoGAP. Panel B corresponds to a BLAST alignment of a tubby candidate gene.
**FIGURE 6** is a block diagram illustrating a first example architecture of a computer system that can be used in connection with examples of the present disclosure.
**FIGURE 7** is a diagram illustrating a computer network that can be used in connection with examples of the present disclosure.
**FIGURE 8** is a block diagram illustrating a second example architecture of a computer system that can be used in connection with examples of the present disclosure.
**FIGURE 9** is a diagram of components of an Immunosignaturing system of the invention.
**FIGURE 10** illustrates exemplary arrays of the invention with distinct peptide densities.
**FIGURE 11** Panel A illustrates a phage display library. Panel B illustrates a peptide microarray.
**FIGURE 12** is a Guitope alignment of Immunosignature peptides to the PA-X frameshift sequence and synthesized tiled peptides. Panel A shows the translated PA-X frameshift sequence with the two peptides from the Immunosignature. Identical amino acids are underlined for the EPME peptide and in bold for the PAMK peptide. Similar amino acid residues are indicated with an asterisks (*) vs a vertical line (|) for identical residues. Panel B shows the tiled peptides with the highlighted residues.
**FIGURE 13** is a graph showing that the predicted epitope is increasingly bound by serum antibodies of mice following an infection with Influenza (H1N1) A/PR/8/34.
**FIGURE 14** is a graphical representation of the outcomes of immunized mice following lethal challenge with Influenza A/PR/8/34. Panel A is a graphical representation of the average daily percent starting weight. Panel B is a graphical representation of the percentage of mice surviving following challenge.
**FIGURE 15** is a graphical representation of traditional measures of immune response. Panel A is a graphical representation of the amount of antigen specific circulating IgG measured for inactive PRB (2006-2007 and 2007-2008 seasonal vaccines by endpoint titer). Panels B through E illustrate the concentrations of IL-2, IL-4, IL-5, INF-g, and IL-12, respectively.
**FIGURE 16** illustrates a comparison of the Immunosignature of live and inactive influenza A/PR/8/34. Panel A is a scatterplot where the live PR8 is on the x-axis and the inactive PR8 is on the y-axis. Panel B is a Venn diagram illustrating the overlap of significantly different peptides. Panel C illustrates the variance in immune response between individuals by PCA analysis. Panel D illustrates the overlap where the first two principal components are plotted and individuals colored by vaccine.

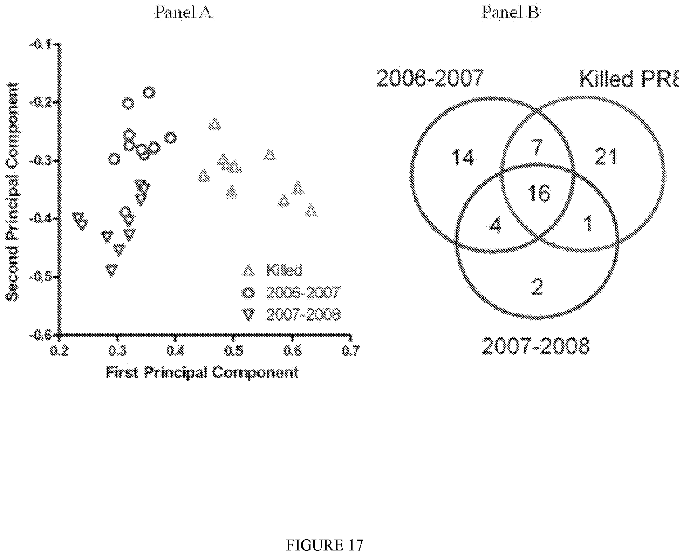

**FIGURE 17** illustrates that an immunosignature can distinguish weaker inactive vaccines from more potent ones. Panel A illustrates variance amongst principal components. Panel B is a Venn diagram illustrating the overlap between the lists.

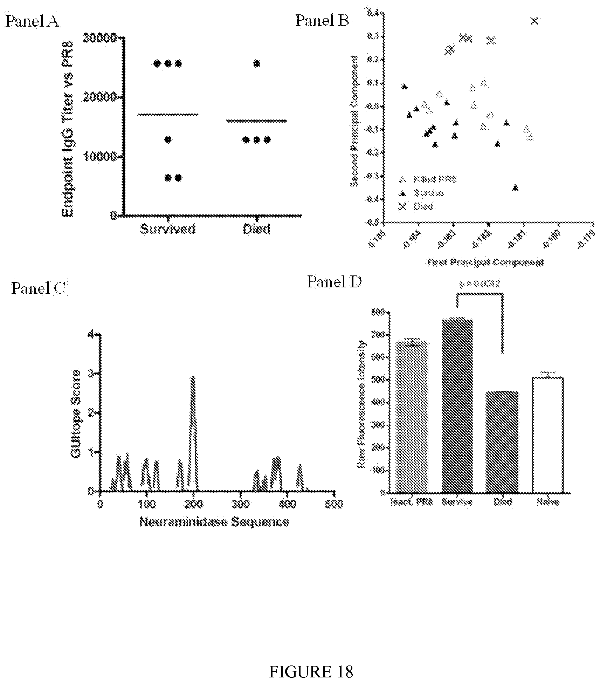

**FIGURE 18** is a graphical representation of data collected in an Immunosignature validation study indicating that antibody cross-reactivity to NA 195-219 was important in protecting seasonal vaccine recipients from the PR8. Panel A illustrates whole virus ELISA endpoint titers for the 2006-2007 vaccine recipients that survived or died following PR8 challenge. Panel B is a graphical representation of the variance among all individuals receiving an inactive vaccine. Panel C is a graphical representation of a GUItope score on the y-axis and amino acid on the x-axis. Panel D is a bar graph of antibody reactivity from pooled sera.
**FIGURE 19** illustrates an interpretation of an Immunosignature on random peptide arrays to determine epitopes recognized by each vaccine.
**FIGURE 20** is an overall schematic of one application of the invention in target identifying screening.

### DETAILED DESCRIPTION

Emerging pathogens, pandemic disease and increasingly antibiotic resistant bacteria are driving the need for new drug and vaccine targets. Searches for new therapeutic targets have employed proteomics or large scale PCR screens, and such technologies are often used to examine differences across drug treatments, diseases, transgenic animals, and others. However, application of those methods to target identifying screening is often limited.

Challenges in drug development targeting, for example, cancer, heart disease, diabetes, pandemic diseases, a plurality of chronic conditions, autoimmune conditions, emerging pathogens, and increasingly antibiotic resistant bacteria are driving the need for new drug and vaccine targets identification. There is an increased interest in the discovery of novel proteins encoded in genomes, for example, the discovery of novel proteins encoded in human pathogens. Nevertheless, existing methods of discovery are not cost-effective, practical, reliable, and/or consistent. Furthermore, there exists a pressing need for the development of assays that can readily characterize newly discovered proteins, for example, by identifying epitopes which bind to those newly developed proteins.

The methods and devices disclosed herein are also capable of personalizing or customizing therapeutic treatment to an individual afflicted with a condition, including cancer, heart disease, diabetes, autoimmune disorders and infections, including pathogenic and microbial infections. Targets against a condition that may be unique, prominent or available to an individual can be identified, and therapeutic treatments developed against the target. For example, targets that may be upregulated or downregulated with a condition may be detected with the methods and devices disclosed herein, particularly where there are genetic polymorphisms or changes in the disease origin. Accordingly, developing therapeutic solutions directed to a specific individual can be achieved with the methods and devices disclosed herein.

The immune system is constantly monitoring for self and nonself proteins, and when encountered, the immune system can amplify the signal from a molecular target that is detected as abnormal by 10¹¹ fold in a week. This suggests that the natural response of the immune system can provide an easily accessible readout of the presence of a molecule in the body of a subject, for example, a novel protein. By comparing the serum immunosignature of healthy and diseased individuals, peptides common to disease can be used in silico to predict potential frameshift or other novel proteins in the diseased cells. The development of peptide arrays of much higher diversity could facilitate this analysis. If made widely available, a general serological reassessment of all pathogens and chronic diseases for novel proteins can be entertained.

The Immunosignaturing microarray is based on complex mixtures of antibodies binding to arrays of peptides. It relies on many-to-many binding of antibodies to the peptides. Each peptide can bind multiple antibodies and each antibody can bind multiple peptides.

A measurement of an activity of an immune system can identify a presence of at least one antibody, an absence of at least one antibody, an upregulation of a plurality of antibodies, and/or a downregulation of a plurality of antibodies. All of those activities can comprise a humoral immune proxy for changes in health which can be measured, characterized, and deciphered with, for example, Immunosignaturing methods.

Immunosignaturing is an array-based technology that quantitates the dynamics of circulating antibodies in a subject. The dynamics of circulating antibodies includes both the presence and the absence of an antibody or a plurality of antibodies from the system of a subject. The method is based on the sensitivity of the antibody profile in an individual to the development of aberrant cells. Even a small number of initiating cancer or other diseased cells can initiate a B-cell response that can be amplified 10¹¹ fold in a week.

In some cases, a method of deciphering an immunosignature comprises the steps of: (a) contacting a peptide array with sera from an individual suffering from a disorder of interest; (b) detecting binding of antibodies in the sera to peptides on the array to generate a disease immune profile; (c) comparing the disease immune profile to a normal control and identifying differentially bound peptides based on one or both of: (i) peptides that bound more antibody in the disease immune profile compared to normal control; and (ii) peptides that bound less antibody in the disease immune profile compared to normal control; wherein proteins corresponding to the differentially bound peptides are therapeutic targets for the disease of interest.

By splaying the antibody repertoire out on an array of peptides (immunosignaturing) and comparing disease to normal control (including, but not limited to non-disease sera contacted with an identical array under the same experimental conditions), the reactive peptides in step (C)(i) and (C)(ii) can be identified to determine the proteins the antibodies are reacting to. For example, the peptides can be identified with informatics methods. In cases where the informatics cannot identify a putative match, such as in the case of discontinuous epitopes, the informative peptide can be used as an affinity reagent to purify reactive antibody. Purified antibody can then be used in standard immunological techniques to identify the target.

Any suitable peptide array can be used on which the peptides are immobilized to a substrate. In some cases, the array comprises between 500-1,000,000 peptides; between 500-500,000 peptides; between 500-250,000 peptides; between 500-100,000 peptides; between 500-50,000 peptides; or between 500-10,000 peptides. In some cases, the peptides are 8-35, 12-35, 15-25, 10-30, or 9-25 amino acids in length. In some cases, the amino acid sequences of the peptides are randomly selected. In some cases, the pattern of amino acids present in the microarray is pre-defined, and the array is not a random peptide array.

In some cases, the amino acid sequences of the peptides have less than 90% sequence identity to known proteins. In some cases, the average spacing between peptides on the array is between about 2-15 nm, about 7-12 nm, about 8-11 nm, about 9 nm, about 2-4 nm, or about 3 nm.

As used herein, the term "substrate" refers to any type of solid support to which the peptides are immobilized. Examples of substrates include, but are not limited to, microarrays; beads; columns; optical fibers; wipes; nitrocellulose; nylon; glass; quartz; diazotized membranes (paper or nylon); silicones; polyformaldehyde; cellulose; cellulose acetate; paper; ceramics; metals; metalloids; semiconductive materials; coated beads; magnetic particles; plastics such as polyethylene, polypropylene, and polystyrene; gel-forming materials; silicates; agarose; polyacrylamides; methylmethracrylate polymers; sol gels; porous polymer hydrogels; nanostructured surfaces; nanotubes (such as carbon nanotubes); and nanoparticles (such as gold nanoparticles or quantum dots). When bound to a substrate, the peptides can be directly linked to the support, or attached to the surface via a linker. Thus, the solid substrate and/or the peptides can be derivatized using methods known in the art to facilitate binding of the peptides to the solid support, so long as the derivitization does not eliminate detection of binding between the peptides and antibodies in the sera.

Other molecules, such as reference or control molecules, can be optionally immobilized on the substrate as well. Methods for immobilizing various types of molecules on a variety of substrates are well known to those of skill in the art. A wide variety of materials can be used for the solid surface. A variety of different materials can be used to prepare the support to obtain various properties. For example, proteins (e.g., bovine serum albumin) or mixtures of macromolecules (e.g., Denhardt's solution) can be used to minimize non-specific binding, simplify covalent conjugation, and/or enhance signal detection.

Sera can be obtained from the individual using techniques known in the art. The individual can be one suffering from any disorder of interest, including but not limited to cancer, diabetes, infection, atherosclerosis, cardiovascular heart disease, stroke, and neurological disorders such as Parkinson's and Alzheimer's.

The peptide array can be contacted with the sera under any suitable conditions to promote binding of antibodies in the sera to peptides immobilized on the array. Thus, the methods of the disclosure are not limited by any specific type of binding conditions employed. Such conditions will vary depending on the array being used, the type of substrate, the density of the peptides arrayed on the substrate, desired stringency of the binding interaction, and nature of the competing materials in the binding solution. In a preferred case, the conditions comprise a step to remove unbound antibodies from the addressable array. Determining the need for such a step, and appropriate conditions for such a step, are well within the level of skill in the art.

Similarly, any suitable detection technique can be used in the methods of the disclosure detecting binding of antibodies in the sera to peptides on the array to generate a disease immune profile; In one case, any type of detectable label can be used to label peptides on the array, including but not limited to radioisotope labels, fluorescent labels, luminescent labels, and electrochemical labels (*i.e*.: ligand labels with different electrode mid-point potential, where detection comprises detecting electric potential of the label). Alternatively, bound antibodies can be detected, for example, using a detectably labeled secondary antibody.

Detection of signal from detectable labels is well within the level of skill in the art. For example, fluorescent array readers are well known in the art, as are instruments to record electric potentials on a substrate (For electrochemical detection see, for example, J. Wang (2000) Analytical Electrochemistry, Vol., 2nd ed., Wiley - VCH, New York).

The control can be any suitable control. In one case, the control comprises non-disease sera contacted with an identical array under the same experimental conditions. Comparison of the disease immune profile to a normal control and identifying differentially bound peptides can be carried out via any suitable technique. As discussed in greater detail in the examples, peptides that (i) bind more antibody in the disease immune profile compared to normal control; and peptides that (ii) bind less antibody in the disease immune profile compared to normal control are therapeutic targets for the disease of interest.

The differentially binding peptides/corresponding protein therapeutic targets of interest can be identified by any suitable method, including but not limited to sequence analysis, comparison to the proteome, prior knowledge of peptides at particular positions on the array, and using the differentially binding as an affinity reagent to purify reactive antibody, which can then be used in standard immunological techniques to identify the target. For example, by comparing the immunosignatures of people with Type I diabetes (TID) to age-matched controls, approximately 200 peptides that bind more antibodies in the Type I diabetes patients were identified (EXAMPLE 1). When these peptides are compared to the human proteome or parts of it, multiple peptides align with proteins known to be up-regulated and released into the extracellular space to cause an autoimmune response in Type I diabetes.

Arrays consisting of peptides corresponding to human protein sequences can be directly aligned with the epitope in the corresponding protein. Random peptide arrays and non-random or pseudo-randoom peptide arrays can be used to identify and align peptide sequences to human proteins. In comparing disease to non-disease samples, a large number of peptides that bind more antibody in the non-disease than the disease samples can be identified.

In comparing disease to non-disease samples a downregulated signal can be specific for a condition. For example, EXAMPLE 1 demonstrates the identification of approximately 400 downregulated peptides in Type I diabetes. When the sequences of these 400 peptides were compared to the human proteins, approximately 70 of the peptides aligned with proteins known to play a role in Type I diabetes. This implies that both the antibody reactivity's which go up and down relative to normal sera are sources of information about relevant proteins in disease. This demonstrates that auto antibodies can be down regulated by the occurrence of disease.

A characteristic feature of the methods of the disclosure is that unique targets for early disease can be identified. Since the immune system can react very early to even a small number of aberrant cells, early targets for treatment can be discovered.

### Multiplexed Detection of Antibody Biomarkers.

Diagnostic approaches designed to detect host-produced antibodies, rather than other less abundant biomarkers, are far more likely to be sufficiently sensitive to detect rare events. A plentiful supply of high-affinity, high-specificity antibodies do not need to be created since a tremendously diverse source of these markers already exists in circulating blood. In multiplexed arrays designed to detect antibodies, panels of protein or peptides are attached to a solid support and then exposed to blood.

Protein arrays are emerging as a high-capacity method capable of simultaneously detecting large numbers of parameters in a single experiment. Protein targets provide a source of conformational epitopes for antibody binding, though linear epitopes are not always exposed. Invitrogen produces one of the more comprehensive protein microarray containing ∼9000 different baculovirus-produced human proteins arrayed onto a single slide. Large-scale potential for these protein arrays is dampened by high costs per slide, lack of scalability, and inconsistencies of recombinant protein production, purification, and stability. Using in vitro synthesized proteins has improved the throughput and success of protein production but inconsistencies in quantities arrayed, stability, post-translational modifications, and biases against membrane (surface), multimeric, and large proteins remain problematic. Both approaches are limited to detecting autoantibodies unless one specifically synthesizes known mutant or pathogen-derived candidate proteins. Biochemical fractionation of diseased cells enables antibodies against modified and mutated antigens to be queried but this is a substantially more complicated procedure (Hanash, S. (2003) Disease proteomics. Nature 422, 226-232).

In contrast to proteins, peptides can be synthesized chemically so that highly reproducible and pure products are available in large quantities, with long shelf lives. Attachment of biologically relevant modifications or detection molecules is simple, and non-natural designs are also possible (Reddy, M.M., et al. Identification of Candidate IgG Biomarkers for Alzheimer's Disease via Combinatorial Library Screening. Cell 144, 132-142).

Peptides are displayed in solution similarly, even when bound to a solid support; therefore, antibody interactions are screened against highly consistent structures regardless of batch to batch production differences. Peptide microarrays have been available far longer than protein microarrays (Panicker, R.C., et al. (2004) Recent advances in peptide-based microarray technologies. Comb Chem High Throughput Screen 7, 547-556), and have been used for a variety of applications. Enzymes (Fu, J., et al. (2010) Exploring peptide space for enzyme modulators. J Am Chem Soc 132, 6419-6424; and Fu, J., et al. (2011) Peptide-modified surfaces for enzyme immobilization. PLoS One 6, e18692), proteins (Diehnelt, C.W., et al. Discovery of high-affinity protein binding ligands-backwards. PLoS One 5, e10728; Greving, M.P., et al. High-throughput screening in two dimensions: binding intensity and off-rate on a peptide microarray. Anal Biochem 402, 93-95; Greving, M.P., et al. Thermodynamic additivity of sequence variations: an algorithm for creating high affinity peptides without large libraries or structural information. PLoS One 5, e15432; Gupta, N., et al. Engineering a synthetic ligand for tumor necrosis factor-alpha. Bioconjug Chem 22, 1473-1478), DNA and small molecules (Boltz, K.W., et al. (2009) Peptide microarrays for carbohydrate recognition. Analyst 134, 650-652; Foong, Y.M., et al. (2012) Current advances in peptide and small molecule microarray technologies. Curr Opin Chem Biol 16, 234-242; Morales Betanzos, C., et al. (2009) Bacterial glycoprofiling by using random sequence peptide microarrays. Chembiochem 10, 877-888), whole cells (Falsey, J.R., et al. (2001) Peptide and small molecule microarray for high throughput cell adhesion and functional assays. Bioconjug Chem 12, 346-353), and antibodies (Cerecedo, I., et al. (2008) Mapping of the IgE and IgG4 sequential epitopes of milk allergens with a peptide microarray-based immunoassay. J Allergy Clin Immunol 122, 589-594; Cretich, M., et al. (2009) Epitope mapping of human chromogranin A by peptide microarrays. Methods Mol Biol 570, 221-232; Lin, J., et al. (2009) Development of a novel peptide microarray for large-scale epitope mapping of food allergens. J Allergy Clin Immunol 124, 315-322, 322 e311-313; Lorenz, P., et al. (2009) Probing the epitope signatures of IgG antibodies in human serum from patients with autoimmune disease. Methods Mol Biol 524, 247-258; Perez-Gordo, M., et al. (2012) Epitope mapping of Atlantic salmon major allergen by peptide microarray immunoassay. Int. Arch Allergy Immunol 157, 31-40; and Shreffler, W.G., et al. (2005) IgE and IgG4 epitope mapping by microarray immunoassay reveals the diversity of immune response to the peanut allergen, Ara h 2. J Allergy Clin Immunol 116, 893-899 are just a subset of the biomolecules that can be assayed for binding to peptides. A classic example is epitope mapping: peptides that span an antigen can be tiled to efficiently decipher the epitope of a monoclonal antibody. A high-specificity antibody will recognize and bind its epitope sequence with little or no measurable binding to other antigen-derived peptides, usually. With this method, different monoclonals raised against the same antigen can be distinguished and characterized.

### Relevance of Cross-Reactivity.

The immune system has evolved to elicit and amplify antibodies that ignore self proteins and bind non-self targets with significant strength. The immune system has evolved to elicit and amplify antibodies that ignore self proteins and bind non-self targets with significant strength. These conflicting pressures become clear at the molecular level. A typical antibody recognizes an epitope of ∼15 amino acids of which ∼5 dominate the binding energy. A change in any of these 5 residues will greatly affect binding strength.

Sequence changes in other epitope positions will alter the spatial conformation of the binding region and modestly affect overall strength. Therefore if binding strength is to be maximized, conditions must be adjusted to permit both high and low affinity residues to interact. This implies a reduced stringency and consequently, allows variants of the epitope sequence to bind an antibody. Further contributing to the potential for cross-reactivity, antibodies have a 50 amino acid variable region that contains many overlapping paratopes (the epitope-recognizing portions of the antibody) (Mohan, S., et al. (2009) Association energetics of cross-reactive and specific antibodies. Biochemistry 48, 1390-1398; Thorpe, I.F., and Brooks, C.L., 3rd (2007) Molecular evolution of affinity and flexibility in the immune system. Proceedings of the National Academy of Sciences of the United States of America 104, 8821-8826; and Zhou, Z.H., et al. (2007) Properties and function of polyreactive antibodies and polyreactive antigen-binding B cells. J Autoimmun. 29, 219-228. Epub 2007 Sep 2020).

Each of these paratopes is comprised of ∼15 amino acids such that paratopes and epitopes are similarly-sized stretches that define complementary regions of shape and charge. A paratope can bind more than one epitope, and a single epitope can bind to more than one paratope, each pair displaying unique binding properties. Since a single antibody carries multiple paratopes, an antibody has a distinct yet potentially diverse set of epitopes that it can bind, with varying strengths. This cross-reactivity and complex interplay of specificity and affinity are hallmarks of a sophisticated immune system that orchestrates a direct attack against an immediate threat and indirect attacks against possible exposure to variants in the future.

In vitro, antibodies specific to a particular linear epitope have been shown not only to bind sequence-related peptides but also unrelated ones (Folgori, A., et al. (1994) A general strategy to identify mimotopes of pathological antigens using only random peptide libraries and human sera. Embo J 13, 2236-2243). These sequence-unrelated peptides, typically showing conformational relatedness, are known as mimotopes and were originally described in early phage display studies (Folgori, A., et al. (1994) A general strategy to identify mimotopes of pathological antigens using only random peptide libraries and human sera. Embo J 13, 2236-2243; Christian, R.B., et al. (1992) Simplified methods for construction, assessment and rapid screening of peptide libraries in bacteriophage. Journal of Molecular Biology 227, 711-718; Liu, R., et al. (2003) Combinatorial peptide library methods for immunobiology research. Experimental Hematology 31, 11-30; Wang, Y., et al. (1995) Detection of Mammary Tumor Virus ENV Gene-like Sequences in Human Breast Cancer. Cancer Research 55, 5173-5179.

Phage-based systems provide the opportunity to build and screen libraries of much larger ligand diversity than possible with most other systems. For example, large random sequence libraries displaying peptides were panned against a particular monoclonal antibody. Iterative rounds of selection often led to the identification of the cognate epitope, but several unrelated peptide sequences as well. The fact that random peptide diversity is many orders of magnitude greater than biological sequence diversity means that the peptides will not correspond to any biological peptide. All binding reactions rely on non-cognate, cross reactivity, an inherent property of antibodies. This implies that a ligand for any category of antibody could be identified: autoantigen, modified antigen, mutated epitope, or non-peptidic mimotope. Despite these advantages to screening in random sequence space, phage display techniques are limited by the repeated rounds of panning with phage and bacterial cultures, a binary selection process, and lack of scalability (Derda, R., et al. (2011) Diversity of phage-displayed libraries of peptides during panning and amplification. Molecules 16, 1776-1803; Szardenings, M. (2003) Phage display of random peptide libraries: applications, limits, and potential. J Recept Signal Transduct Res 23, 307-34953, 54). To date, random phage libraries have not yielded an antibody biomarker.

### Immunosignaturing.

Immunosignaturing is a synthesis of the technologies described above. First, rather than display peptides biologically on a phage, linking synthetic and longer peptides onto a glass slide in addressable ordered arrays is a far more systematic method. Although phage libraries can exceed 10¹⁰ individual clones, microarrays have increased from a few thousand to millions of spots per slide. The cost, reliability, precision, and assay speed imbue microarrays with significant advantages. Microarrays have proven themselves invaluable for genomics and proteomics due to their low cost and scalability and commercial array chambers and scanners have existed for years.

Second, using antibodies as biomarkers of disease takes advantage of a stable and easily accessible molecule and the immune system's convenient properties of diversity, surveillance, and biological amplification. The complexity of a mammalian immune system is staggering (Janeway, C., and Travers, J. (1997) Immunobiology: The Immune System in Health and Disease. Current Biology Limited) and therefore so is the information content. As immunologists explore the immunome there is growing consensus that the antibody repertoire, capable of >10¹⁰ different molecular species (Nobrega, A., et al. (1998) Functional diversity and clonal frequencies of reactivity in the available antibody repertoire. European Journal of Immunology 28, 1204-1215), is a dynamic database of past, current, and even prodromic perturbations to an individual's health status.

Third, use of random sequence peptides enables the diversity of the antibody repertoire to be matched by an unbiased, comprehensive library of ligands to screen. Random-sequence peptides can be used in phage display libraries, but they carry biases and are not in an unordered, poorly controlled format. Since random peptide sequences have no constraints and no intentional homology to biological space, the microarrays contain sparse but very broad coverage of sequence space. Normal, mutated, post-translationally modified, and mimetic epitopes corresponding to any disease or organism can be screened on the same microarray. Recent publications in the field have used 10,000 unique random-sequence 20-mer peptides to characterize a multitude of disease states 1, 10, (Brown, J.R., et al. (2011) Statistical methods for analyzing immunosignatures. BMC Bioinformatics 12, 349; Hughes, A.K., et al. (2012) Immunosignaturing can detect products from molecular markers in brain cancer. PLoS One 7, e40201; Kroening, K., et al. (2012) Autoreactive antibodies raised by self derived de novo peptides can identify unrelated antigens on protein microarrays. Are autoantibodies really autoantibodies? Exp Mol Pathol 92, 304-311; Kukreja, M., et al. (2012) Comparative study of classification algorithms for immunosignaturing data. BMC Bioinformatics 13, 139; Kukreja, M., et al. (2012) Immunosignaturing Microarrays Distinguish Antibody Profiles of Related Pancreatic Diseases. Journal of Proteomics and Bioinformatics; and Legutki, J.B., et al. (2010) A general method for characterization of humoral immunity induced by a vaccine or infection. Vaccine 28, 4529-4537).

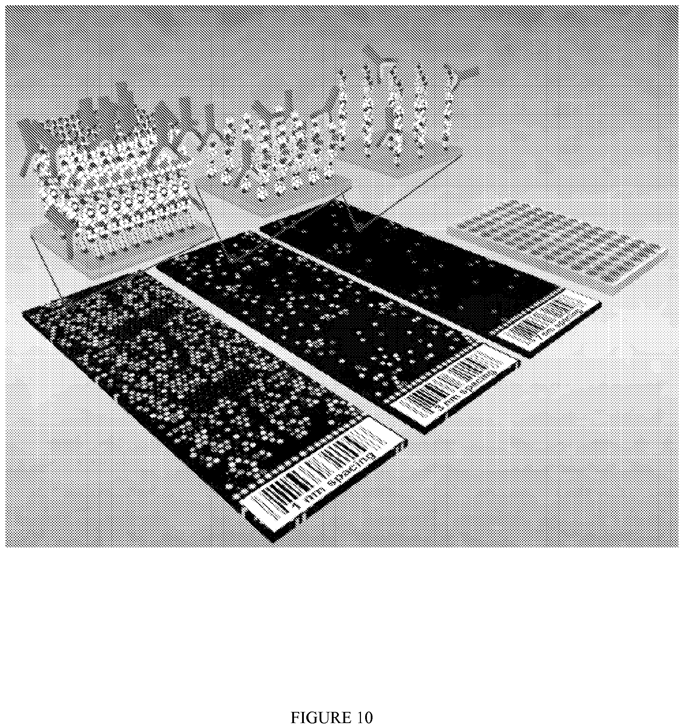

There are several notable differences in the results obtained from phage display versus immunosignaturing microarrays. Immunosignaturing queries all of the peptides on the array and produces binding values for each. Phage display yields sequences that survive restrictive selection, and typically identifies only consensus sequences. Processing immunosignaturing microarrays takes hours rather than weeks. **FIGURE 10** displays the distinction between these technologies. Technically an 'immunosignature' refers to the statistically significant pattern of peptides, each with specific binding values that can robustly classify one state of disease from others.

This integration of technologies can represent progress toward the goal of a universally applicable early diagnostic platform. The key issues remaining to be addressed are whether or not: i) the immune system elicits consistent disease-specific humoral responses to both infectious and chronic diseases, ii) antibodies respond sufficiently early to in the etiology of disease to be clinically useful and iii) the assay is sufficiently sensitive, informative, inexpensive, and scalable to screen large numbers of patient samples for confident determinations. If these points can be satisfied, then the immunosignature of any immune-related disease can be discovered. These defined patterns of reactivity can then be used to diagnose disease early and comprehensively. If these tests can be made widely accessible to the population, immunosignaturing could form the basis for a long-term health monitoring system with important implications at individual but also epidemiological levels. We present several features of the platform that are promising in this regard.

Immunosignaturing is a synthesis of the technologies described above. First, rather than display peptides biologically on a phage, linking synthetic and longer peptides onto a glass slide in addressable ordered arrays is a far more systematic method. Although phage libraries can exceed 10¹⁰ individual clones, microarrays have increased from a few thousand to millions of spots per slide. The cost, reliability, precision, and assay speed imbue microarrays with significant advantages. Microarrays have proven themselves invaluable for genomics and proteomics due to their low cost and scalability and commercial array chambers and scanners have existed for years. Second, using antibodies as biomarkers of disease takes advantage of a stable and easily accessible molecule and the immune system's convenient properties of diversity, surveillance, and biological amplification.

The complexity of a mammalian immune system is staggering and therefore so is the information content. As immunologists explore the immunome there is growing consensus that the antibody repertoire, capable of >10¹⁰ different molecular species, is a dynamic database of past, current, and even prodromic perturbations to an individual's health status. Third, use of random sequence peptides enables the diversity of the antibody repertoire to be matched by an unbiased, comprehensive library of ligands to screen. Random-sequence peptides can be used in phage display libraries, but they carry biases and are not in an unordered, poorly controlled format. Since random peptide sequences have no constraints and no intentional homology to biological space, the microarrays contain sparse but very broad coverage of sequence space.

Normal, mutated, post-translationally modified, and mimetic epitopes corresponding to any disease or organism can be screened on the same microarray. Publications in the field have used 10,000 unique random-sequence 20-mer peptides to characterize a multitude of disease states. There are several notable differences in the results obtained from phage display versus immunosignaturing microarrays. Immunosignaturing queries all of the peptides on the array and produces binding values for each. Phage display yields sequences that survive restrictive selection, and typically identifies only consensus sequences.

Processing immunosignaturing microarrays can take hours rather than weeks. An 'immunosignature' refers to the statistically significant pattern of peptides, each with specific binding values that can robustly classify one state of disease from others. Accordingly, one aspect of the cases disclosed herein is the relatively quick processing time for querying an immunosignature array with a complex biological sample, wherein the querying and processing time can take up to 10 minutes, up to 20 minutes, up to 30 minutes, up to 45 minutes, up to 60 minutes, up to 90 minutes, up to 2 hours, up to 3 hours, up to 4 hours or up to 5 hours. Alternatively, the querying and processing time can take not more than 10 minutes, not more than 20 minutes, not more than 30 minutes, not more than 45 minutes, not more than 60 minutes, not more than 90 minutes, not more than 2 hours, not more than 3 hours, not more than 4 hours or not more than 5 hours.

This integration of technologies can represent progress toward the goal of a universally applicable early diagnostic platform. The key issues remaining to be addressed are whether or not: i) the immune system elicits consistent disease-specific humoral responses to both infectious and chronic diseases, ii) antibodies respond sufficiently early to in the etiology of disease to be clinically useful and iii) the assay is sufficiently sensitive, informative, inexpensive, and scalable to screen large numbers of patient samples for confident determinations. If these points can be satisfied, then the immunosignature of any immune-related disease can be discovered.

These defined patterns of reactivity can then be used to diagnose disease early and comprehensively. If these tests can be made widely accessible to the population, immunosignaturing could form the basis for a long-term health monitoring system with important implications at individual but also epidemiological levels. We present several features of the platform that are promising in this regard.

### Unique Features of Immunosignaturing.

In addition to the affinity of an antibody's paratope for the ligand, binding strength can be influenced by the concentration of the antibody species in serum. Unlike phage display, immunosignaturing can quantitatively measure the product of these parameters, and can do so with a very large dynamic range (Legutki, J.B., et al. (2010) A general method for characterization of humoral immunity induced by a vaccine or infection. Vaccine 28, 4529-4537; Stafford, P., and Johnston, S. (2011) Microarray technology displays the complexities of the humoral immune response. Expert Rev Mol Diagn 11, 5-8; Halperin, R.F., et al. (2011) Exploring Antibody Recognition of Sequence Space through Random-Sequence Peptide Microarrays. Molecular & Cellular Proteomics 10).

Scientists used this capability to examine the binding of high affinity monoclonal antibodies to the immunosignaturing microarrays. They found that a single monoclonal recognized hundreds of random sequences, and the varying strengths of these unique binding reactions could be measured and compared (Halperin, R.F., et al. (2011) Exploring Antibody Recognition of Sequence Space through Random-Sequence Peptide Microarrays. Molecular & Cellular Proteomics 10). Curiously, many of these off-target mimotope interactions had higher binding than the cognate epitope. Although the corresponding solution-phase binding of these interactions is low, the way the immunosignaturing microarray is constructed enhances these interactions. This immunological phenomenon of off-target antibody binding to the immunosignaturing microarray is central to the technology.

Another important observation is the greater sensitivity of immunosignaturing for the detection of low affinity interactions than either phage display or ELISA-based assays (Stafford, P., and Johnston, S. (2011) Microarray technology displays the complexities of the humoral immune response. Expert Rev Mol Diagn 11). The high sensitivity is a consequence of the high density of peptides on the slide surface and has been called the "immunosignaturing effect". This has been established by printing and testing different spatial arrangements of peptides on the functionalized glass surface. If arrays are printed such that peptides are spaced about 9 to about 12 nm apart, cognate epitopes compete for antibodies more favorably than the off-target random peptides (with the exception of very strong mimotopes).

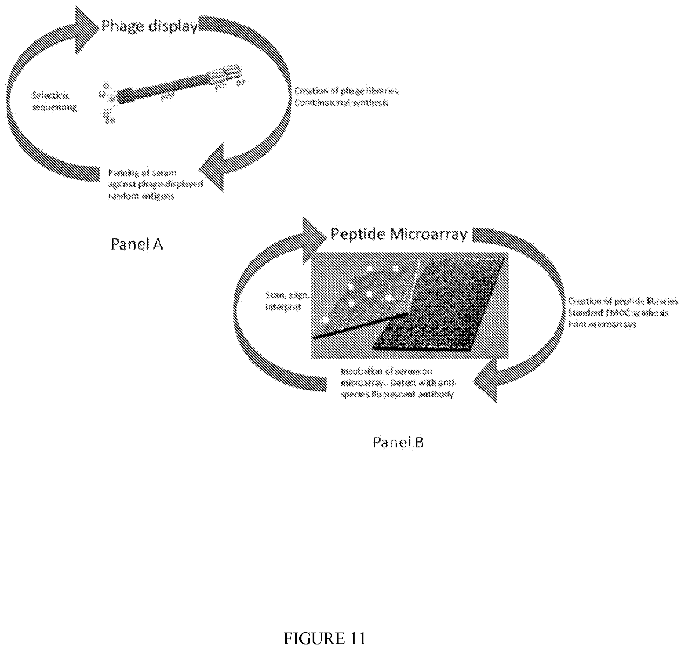

We commonly space peptides 1-2 nm apart on average but observe the off-target binding with peptides spaced 3-4 nm apart. If the peptides are spaced from about 1 to about 1.5 nm apart, then an increase in off-target binding is observed. Tightly packed peptides appear to trap antibodies through avidity and rapid rebinding. This concept has been shown to be extremely reproducible, and is illustrated in **FIGURE 11** (Stafford, P., et al. (2012) Physical characterization of the "immunosignaturing effect". Mol Cell Proteomics 11, M111 011593; Chase, B.A., et al. (2012) Evaluation of biological sample preparation for immunosignature-based diagnostics. Clin Vaccine Immunol 19, 352-358; Hughes, A.K., et al. (2012) Immunosignaturing can detect products from molecular markers in brain cancer. PLoS One 7, e40201; Restrepo, L., et al. (2011) Application of immunosignatures to the assessment of Alzheimer's disease. Annals of Neurology 70, 286-295). While the sequences of the peptides are entirely random, their off-target captures of antibody are clearly not; rather, the patterns of sera binding to the array are remarkably coherent. An early concern relative to this technology was that the large diversity of antibody species in any serum sample might lead to overlapping binding competitions resulting in a flat, uninformative field of intensities. The data have not borne this out. In fact even a purified monoclonal antibody diluted into serum retains its distinct reactivity pattern with little to no loss of binding (Uhlen, M., and Hober, S. (2009) Generation and validation of affinity reagents on a proteome-wide level. J Mol Recognit 22, 57-64).

Classical statistical models used to explain conventional nucleic acid microarrays (Draghici, S. (2012) Statistics and Data Analysis for Microarrays Using R and Bioconductor. Chapman & Hall/CRC) do not have the flexibility to address the new complexities presented by immunosignature arrays. Rather than a one-to-one binding model that describes RNA or DNA binding to complimentary probes on a microarray, the immunosignaturing peptides can bind to more than one antibody, and many different antibodies can bind to the same peptide. Three different reports compared methods for image analysis (Yang, Y., et al. (2011) Segmentation and intensity estimation for microarray images with saturated pixels. BMC Bioinformatics 12, 462), factor analysis and mixture models (Brown, J.R., et al. (2011) Statistical methods for analyzing immunosignatures. BMC Bioinformatics 12, 349), and classification (Kukreja, M., et al. (2012) Comparative study of classification algorithms for immunosignaturing data. BMC Bioinformatics 13, 139) specifically for immunosignaturing. There are a number of fundamental properties of the immunosignaturing microarray that enable discriminating diseases.

First, control sera from healthy volunteers display a rather broad distribution of baseline binding reactivity. This imposes a requirement that a large-scale study using the technology must sample sera from a large number of non-diseased individuals to accommodate the population variability. Second, signatures from sera of persons with a given disease are extremely consistent, unlike that of the non-disease sera. This observation implies that the immune system is constantly probing and reacting to local environments causing broad differences in signatures. However, once directed toward an antigen, antibodies tend to form a narrow and well-defined signature with little individual variability.

Even so, the technology is able to discern sub-types of disease (Hughes, A.K., et al. (2012) Immunosignaturing can detect products from molecular markers in brain cancer. PLoS One 7, e40201) while still providing a distinction between controls and affected. The analysis of common relationships and covariances between pluralities of peptides provides tremendous discerning power that is not possible at the single epitope level. Immunologically, the antibody: peptide binding patterns are not created by a non-specific danger signal or the activities of natural antibodies: they are created by a recognizable stimulus. Antibody adsorption experiments demonstrated that the peptides from an influenza infection bind mostly virus-specific antibodies and the signature of Alzheimer's Disease binds many anti-Aβ antibodies (Legutki, J.B., et al. (2010) A general method for characterization of humoral immunity induced by a vaccine or infection. Vaccine 28, 4529-4537; Restrepo, L., et al. (2011) Application of immunosignatures to the assessment of Alzheimer's disease. Annals of Neurology 70, 286-295).

The disease determinations by immunosignaturing have correlated well with the results obtained using current diagnostic tests (Hughes, A.K., et al. (2012) Immunosignaturing can detect products from molecular markers in brain cancer. PLoS One 7, e40201; Kukreja, M., et al. (2012) Immunosignaturing Microarrays Distinguish Antibody Profiles of Related Pancreatic Diseases. Journal of Proteomics and Bioinformatics; Legutki, J.B., et al. (2010) A general method for characterization of humoral immunity induced by a vaccine or infection. Vaccine 28, 4529-4537). Immunosignatures carry historical health information not accessible with traditional diagnostics; namely, both immediate and memory responses can be detected (Legutki, J.B., et al. (2010) A general method for characterization of humoral immunity induced by a vaccine or infection. Vaccine 28, 4529-4537). To date the approach has been applied to more than 33 different diseases and sequelae including viral, bacterial, fungal and parasitic infections, cancers, diabetes, autoimmune disease, transplant patients and many chronic diseases in mouse, rat, dog, pig, and human hosts. A highly reproducible pattern of peptide binding patterns can be established that correlates with pathology.

These binding profiles correctly classify blinded sera samples obtained from patients and healthy volunteers and outperform classic immunological tests in sensitivity and accuracy. In a large-scale study, immunosignatures were able to diagnose Valley Fever patients with very high accuracy, including the correct diagnosis of patients that were initially negative by standard ELISA tests. Analyses of patient immunosignatures were able to distinguish among and within cancers (Brown, J.R., et al. (2011) Statistical methods for analyzing immunosignatures. BMC Bioinformatics 12, 349; Hughes, A.K., et al. (2012) Immunosignaturing can detect products from molecular markers in brain cancer. PLoS One 7, e40201; Kukreja, M., et al. (2012) Immunosignaturing Microarrays Distinguish Antibody Profiles of Related Pancreatic Diseases. Journal of Proteomics and Bioinformatics; and Yang, Y., et al. (2011) Segmentation and intensity estimation for microarray images with saturated pixels. BMC Bioinformatics 12, 462) even to the point of accurately diagnosing cancer types that will and will not respond to drug treatment (Hughes, A.K., et al. Immunosignaturing can detect products from molecular markers in brain cancer. PLoS One 7, e40201).

One of the most unique features of the immunosignaturing technology can turn out to be measurement of decreases in particular peptide:antibody reactivity, a class of interactions previously not measurable. Namely, while sera from diseased individuals produce high signals relative to normal sera, there are also peptides that consistently show reduced binding relative to healthy persons. (Kukreja, M., et al. (2012) Immunosignaturing Microarrays Distinguish Antibody Profiles of Related Pancreatic Diseases. Journal of Proteomics and Bioinformatics; and Legutki, J.B., et al. (2010), A general method for characterization of humoral immunity induced by a vaccine or infection. Vaccine 28, 4529-4537). The role of these "down" peptides in an immune response is intriguing. Although at its simplest level, these "down" peptides enhance disease classification, there can be some underlying immunological phenomenon that would not otherwise be seen.

### Binding of Molecules to an Array.

According to the National Cancer Institute, there are approximately 150 classes of cancer and, depending on how one defines them, hundreds of distinct subtypes. Antibodies are often raised against antigens expressed by tumor cells, and are subsequently amplified during B-cell maturation. Antibodies are also raised during a response to a vaccine or infection. Antibodies can also be raised during the daily exposure of a subject to various pathogenic, as well as non-pathogenic stimuli.

The process of antibody amplification in a subject's body can generate an ample supply of subject specific markers associated with a condition. Antibody amplification can provide ample numbers of antibodies which are associated with a specific health state of a subject and/or a condition. The presence of a sufficient number of antibodies in a sample can reduce a requirement for artificial biomarker amplification in a method of health monitoring. The presence of a sufficient number of antibodies in a sample can allow a small quantity of sample to be successfully applied in, for example, a method of health monitoring.

The methods and arrays of the disclosure allow for, for example, methods of identifying a therapeutic target, vaccine, or screening for therapeutic targets against a cancer and/or immune disorder with small quantitites of biological samples from a subject. In some cases, the biological samples can be used in a method of the disclosure without further processing and in small quantities. In some cases, the biological samples comprise, blood, serum, saliva, sweat, cells, tissues, or any bodily fluid. In some cases, about 0.5 nl, about 1 nl, about 2 nl, about 3 nl, about 4 nl, about 5 nl, about 6 nl, about 7 nl, about 8 nl, about 9 nl, about 10 nl, about 11 nl, about 12 nl, about 13 nl, about 14 nl, about 15 nl, about 16 nl, about 17 nl, about 18 nl, about 19 nl, about 20 nl, about 21 nl, about 22 nl, about 23 nl, about 24 nl, about 25 nl, about 26 nl, about 27 nl, about 28 nl, about 29 nl, about 30 nl, about 31 nl, about 32 nl, about 33 nl, about 34 nl, about 35 nl, about 36 nl, about 37 nl, about 38 nl, about 39 nl, about 40 nl, about 41 nl, about 42 nl, about 43 nl, about 44 nl, about 45 nl, about 46 nl, about 47 nl, about 48 nl, about 49 nl, or about 50 nl, about 51 nl, about 52 nl, about 53 nl, about 54 nl, about 55 nl, about 56 nl, about 57 nl, about 58 nl, about 59 nl, about 60 nl, about 61 nl, about 62 nl, about 63 nl, about 64 nl, about 65 nl, about 66 nl, about 67 nl, about 68 nl, about 69 nl, about 70 nl, about 71 nl, about 72 nl, about 73 nl, about 74 nl, about 75 nl, about 76 nl, about 77 nl, about 78 nl, about 79 nl, about 80 nl, about 81 nl, about 82 nl, about 83 nl, about 84 nl, about 85 nl, about 86 nl, about 87 nl, about 88 nl, about 89 nl, about 90 nl, about 91 nl, about 92 nl, about 93 nl, about 94 nl, about 95 nl, about 96 nl, about 97 nl, about 98 nl, about 99 nl, about 0.1, about 0.2 µl, about 0.3 µl, about 0.4 µl, about 0.5 µl, about 0.6 µl. about 0.7 µl, about 0.8 µl, about 0.9 µl, about 1 µl, about 2 µl, about 3 µl, about 4 µl, about 5 µl, about 6 µl, about 7 µl, about 8 µl, about 9 µl, about 10 µl, about 11 µl, about 12 µl, about 13 µl, about 14 µl, about 15 µl, about 16 µl, about 17 µl, about 18 µl, about 19 µl, about 20 µl, about 21 µl, about 22 µl, about 23 µl, about 24 µl, about 25 µl, about 26 µl, about 27 µl, about 28 µl, about 29 µl, about 30 µl, about 31 µl, about 32 µl, about 33 µl, about 34 µl, about 35µl, about 36 µl, about 37 µl, about 38 µl, about 39 µl, about 40 µl, about 41 µl, about 42 µl, about 43 µl, about 44 µl, about 45 µl, about 46 µl, about 47 µl, about 48 µl, about 49 µl, or about 50 µl of biological samples are required for analysis by an array and method of the disclosure.

A biological sample from a subject can be for example, collected from a subject and directly contacted with an array of the disclosure. In some cases, the biological sample does not require a preparation or processing step prior to being contacted with an array of the disclosure. In some cases, a dry blood sample from a subject is reconstituted in a dilution step prior to being contacted with an array of the disclosure. A dilution can provide an optimum concentration of an antibody from a biological sample of a subject for immunosignaturing.

The methods and arrays of the disclosure allow the identification of a therapeutic target, a vaccine, or a screening for therapeutic targets against a cancer and/or an immune disorder with small quantities of biological samples from a subject. In some cases, the methods of the disclosure require no more than about 0.5 nl to about 50 nl, no more than about 1 nl to about 100 nl, no more than about 1 nl to about 150 nl, no more than about 1 nl to about 200 nl, no more than about 1 nl to about 250 nl, no more than about 1 nl to about 300 nl, no more than about 1 nl to about 350 nl, no more than about 1 nl to about 400 nl, no more than about 1 to about 450 nl, no more than about 5 nl to about 500 nl, no more than about 5 nl to about 550 nl, no more than about 5 nl to about 600 nl, no more than about 5 nl to about 650 nl, no more than about 5 nl to about 700 nl, no more than about 5 nl to about 750 nl, no more than about 5 nl to about 800 nl, no more than about 5 nl to about 850 nl, no more than about 5 nl to about 900 nl, no more than about 5 nl to about 950 nl, no more than about 5 nl to about 1 µl, no more than about 0.5 µl to about 1 µl, no more than about 0.5 µl to about 5 µl, no more than about 1 µl to about 10 µl, no more than about 1 µl to about 20 µl, no more than about 1 µl to about 30 µl, no more than about 1 µl to about 40 µl, or no more than about 1 µl to about 50 µl.

In some cases, the methods of the disclosure require at least 0.5 nl to about 50 nl, at least about 1 nl to about 100 nl, at least about 1 nl to about 150 nl, at least about 1 nl to about 200 nl, at least about 1 nl to about 250 nl, at least about 1 nl to about 300 nl, at least about 1 nl to about 350 nl, at least about 1 nl to about 400 nl, at least about 1 to about 450 nl, at least about 5 nl to about 500 nl, at least about 5 nl to about 550 nl, at least about 5 nl to about 600 nl, at least about 5 nl to about 650 nl, at least about 5 nl to about 700 nl, at least about 5 nl to about 750 nl, at least about 5 nl to about 800 nl, at least about 5 nl to about 850 nl, at least about 5 nl to about 900 nl, at least about 5 nl to about 950 nl, at least about 5 nl to about 1 µl, at least about 0.5 µl to about 1 µl, at least about 0.5 µl to about 5 µl, at least about 1 µl to about 10 µl, at least about 1 µl to about 20 µl, at least about 1 µl to about 30 µl, at least about 1 µl to about 40 µl, at least about 1 µl to about 50 µl, or at least 50 µl

A subject can provide a plurality of biological sample, including a solid biological sample, from for example, a biopsy or a tissue. In some cases, about 1 mg, about 5 mgs, about 10 mgs, about 15 mgs, about 20 mgs, about 25 mgs, about 30 mgs, about 35 mgs, about 40 mgs, about 45 mgs, about 50 mgs, about 55 mgs, about 60 mgs, about 65 mgs, about 7 mgs, about 75 mgs, about 80 mgs, about 85 mgs, about 90 mgs, about 95 mgs, or about 100 mgs of biological sample are required by an array and method of the disclosure.

In some cases, no more than about 1 mg to about 5 mgs, no more than about 1 mg to about 10 mgs, no more than about 1 mg to about 20 mgs, no more than about 1 mg to about 30 mgs, no more than about 1 mg to about 40 mgs, no more than about 1 mg to about 50 mgs, no more than about 50 mgs to about 60 mgs, no more than about 50 mgs to about 70 mgs, no more than about 50 mgs to about 80 mgs, no more than about 50 mgs to about 90 mgs, no more than about 50 mgs to about 100 mgs of biological sample are required by the methods and arrays of the disclosure.

In some cases, at least about 1 mg to about 5 mgs, at least about 1 mg to about 10 mgs, at least about 1 mg to about 20 mgs, at least about 1 mg to about 30 mgs, at least about 1 mg to about 40 mgs, at least about 1 mg to about 50 mgs, at least about 50 mgs to about 60 mgs, at least about 50 mgs to about 70 mgs, at least about 50 mgs to about 80 mgs, at least about 50 mgs to about 90 mgs, at least about 50 mgs to about 100 mgs of biological sample are required by the methods and arrays of the disclosure.

The methods and arrays of the disclosure provide sensitive methods for the identification of a therapeutic target, and/or a vaccine. The methods and arrays of the disclosure provide sensitive methods for a screening of therapeutic targets against a cancer and/or immune disorder of conditions with small quantities of biological samples from a subject. In some cases, biological samples from a subject are too concentrated and require a dilution prior to being contacted with an array of the disclosure. A plurality of dilutions can be applied to a biological sample prior to contacting the sample with an array of the disclosure. A dilution can be a serial dilution, which can result in a geometric progression of the concentration in a logarithmic fashion. For example, a ten-fold serial dilution can be 1 M, 0.01 M, 0.001 M, and a geometric progression thereof. A dilution can be, for example, a one-fold dilution, a two-fold dilution, a three-fold dilution, a four-fold dilution, a five-fold dilution, a six-fold dilution, a seven-fold dilution, an eight-fold dilution, a nine-fold dilution, a ten-fold dilution, a sixteen-fold dilution, a twenty-five-fold dilution, a thirty-two-fold dilution, a sixty-four-fold dilution, and/or a one-hundred-and-twenty-five-fold dilution.

A biological sample can be derived from a plurality of sources within a subject's body and a biological sample can be collected from a subject in a plurality of different circumstances. A biological sample can be collected, for example, during a routine medical consultation, such as a blood draw during an annual physical examination. A biological sample can be collected during the course of a non-routine consultation, for example, a biological sample can be collected during the course of a biopsy. A subject can also collect a biological sample from oneself, and a subject can provide a biological sample to be analyzed by the methods and systems of the invention in a direct-to-consumer fashion. In some cases, a biological sample can be mailed to a provider of the methods and arrays of the invention. In some cases, a dry biological sample, such as a dry blood sample from a subject on a filter paper, is mailed to a provider of the methods and arrays of the invention.

The binding of a molecule to an array of the invention creates a pattern of binding that can be associated with a condition. The affinity of binding of a molecule to a peptide in the array can be mathematically associated with a condition. The off-target binding pattern of an antibody to a plurality of different peptides of the invention can be mathematically associated with a condition. The avidity of binding of a molecule to a plurality of different peptides of the invention can be mathematically associated with a condition. The off-target binding and avidity can comprise the interaction of a molecule in a biological sample with multiple, non-identical peptides in a peptide array. An avidity of binding of a molecule with multiple, non-identical peptides in a peptide array can determine an association constant of the molecule to the peptide array. In some cases, the concentration of an antibody in a sample contributes to an avidity of binding to a peptide array, for example, by trapping a critical number or antibodies in the array and allowing for rapid rebinding of an antibody to an array.

The avidity of binding of biological molecules to an array can be determined by a combination of multiple bond interactions. A cross-reactivity of an antibody to multiple peptides in a peptide array can contribute to an avidity of binding. In some cases, an antibody can recognize an epitope of about 3 amino acids, about 4 amino acids, about 5 amino acids, about 6 amino acids, about 7 amino acids, about 8 amino acids, about 9 amino acids, about 10 amino acids, about 11 amino acids, about 12 amino acids, about 13 amino acids, about 14 amino acids, about 15 amino acids, about 16 amino acids, or about 17 amino acids. In some cases, a sequence of about 5 amino acids dominates a binding energy of an antibody to a peptide.

An off-target binding, and/or avidity, of a molecule to an array of the invention can, for example, effectively compress binding affinities that span femtomolar (fM) to micromolar (µM) dissociation constants into a range that can be quantitatively measured using only 3 logs of dynamic range. A molecule can bind to a plurality of peptides in the array with association constants of 10³ M⁻¹ or higher. A molecule can bind to a plurality of peptides in the array with association constants ranging from 10³ to 10⁶ M⁻¹, 2 x 10³ M⁻¹ to 10⁶ M⁻¹, and/or association constants ranging from 10⁴ M⁻¹ to 10⁶ M⁻¹. A molecule can bind to a plurality of peptides in the array with a dissociation constant of about 1 fM, about 2 fM, about 3 fM, about 4 fM, about 5 fM, about 6 fM, about 7 fM, about 8 fM, about 9 fM, about 10 fM, about 20 fM, about 30 fM, about 40 fM, about 50 fM, about 60 fM, about 70 fM, about 80 fM, about 90 fM, about 100 fM, about 200 fM, about 300 fM, about 400 fM, about 500 fM, about 600 fM, about 700 fM, about 800 fM, about 900 fM, about 1 picomolar (pM), about 2 pM, about 3 pM, about 4 pM, about 5 pM, about 6 pM, about 7 pM, about 8 pM, about 9 pM, about 10 pM, about 20 pM, about 30 pM, about 40 pM, about 50 pM, about 60 pM, about 7 pM, about 80 pM, about 90 pM, about 100 pM, about 200 pM, about 300 pM, about 400 pM, about 500 pM, about 600 pM, about 700 pM, about 800 pM, about 900 pM, about 1 nanomolar (nM), about 2 nM, about 3 nM, about 4 nM, about 5 nM, about 6 nM, about 7 nM, about 8 nM, about 9 nM, about 10 nM, about 20 nM, about 30 nM, about 40 nM, about 50 nm, about 60 nM, about 70 nM, about 80 nM, about 90 nM, about 100 nM, about 200 nM, about 300 nM, about 400 nM, about 500 nM, about 600 nM, about 700 nM, about 800 nM, about 900 nM, about 1 µM, about 2 µM, about 3 µM, about 4 µM, about 5 µM, about 6 µM, about 7 µM, about 8 µM, about 9 µM, about 10 µM, about 20 µM, about 30 µM, about 40 µM, about 50 µM, about 60 µM, about 70 µM, about 80 µM, about 90 µM, or about 100 µM.

A molecule can bind to a plurality of peptides in the array with a dissociation constant of at least 1 fM, at least 2 fM, at least 3 fM, at least 4 fM, at least 5 fM, at least 6 fM, at least 7 fM, at least 8 fM, at least 9 fM, at least 10 fM, at least 20 fM, at least 30 fM, at least 40 fM, at least 50 fM, at least 60 fM, at least 70 fM, at least 80 fM, at least 90 fM, at least 100 fM, at least 200 fM, at least 300 fM, at least 400 fM, at least 500 fM, at least 600 fM, at least 700 fM, at least 800 fM, at least 900 fM, at least 1 picomolar (pM), at least 2 pM, at least 3 pM, at least 4 pM, at least 5 pM, at least 6 pM, at least 7 pM, at least 8 pM, at least 9 pM, at least 10 pM, at least 20 pM, at least 30 pM, at least 40 pM, at least 50 pM, at least 60 pM, at least 7 pM, at least 80 pM, at least 90 pM, at least 100 pM, at least 200 pM, at least 300 pM, at least 400 pM, at least 500 pM, at least 600 pM, at least 700 pM, at least 800 pM, at least 900 pM, at least 1 nanomolar (nM), at least 2 nM, at least 3 nM, at least 4 nM, at least 5 nM, at least 6 nM, at least 7 nM, at least 8 nM, at least 9 nM, at least 10 nM, at least 20 nM, at least 30 nM, at least 40 nM, at least 50 nm, at least 60 nM, at least 70 nM, at least 80 nM, at least 90 nM, at least 100 nM, at least 200 nM, at least 300 nM, at least 400 nM, at least 500 nM, at least 600 nM, at least 700 nM, at least 800 nM, at least 900 nM, at least 1 µM, at least 2 µM, at least 3 µM, at least 4 µM, at least 5 µM, at least 6 µM, at least 7 µM, at least 8 µM, at least 9 µM, at least 10 µM, at least 20 µM, at least 30 µM, at least 40 µM, at least 50 µM, at least 60 µM, at least 70 µM, at least 80 µM, at least 90 µM, or about 100 µM.

A dynamic range of binding of an antibody from a biological sample to a peptide microarray can be described as the ratio between the largest and smallest value of a detected signal of binding. A signal of binding can be, for example, a fluorescent signal detected with a secondary antibody. Traditional assays are limited by pre-determined and narrow dynamic ranges of binding. The methods and arrays of the invention can detected a broad dynamic range of antibody binding to the peptides in the array of the invention. In some cases, a broad dynamic range of antibody binding can be detected on a logarithmic scale. In some cases, the methods and arrays of the invention allow the detection of a pattern of binding of a plurality of antibodies to an array using up to 2 logs of dynamic range, up to 3 logs of dynamic range, up to 4 logs of dynamic range or up to 5 logs of dynamic range.

The composition of molecules in an array can determine an avidity of binding of a molecule to an array. A plurality of different molecules can be present in an array used in the prevention, treatment, diagnosis or monitoring of a health condition. Non-limiting examples of biomolecules include amino acids, peptides, peptide-mimetics, proteins, recombinant proteins antibodies (monoclonal or polyclonal), antibody fragments, antigens, epitopes, carbohydrates, lipids, fatty acids, enzymes, natural products, nucleic acids (including DNA, RNA, nucleosides, nucleotides, structure analogs or combinations thereof), nutrients, receptors, and vitamins. In some cases, a molecule in an array is a mimotope, a molecule that mimics the structure of an epitope and is able to bind an epitope-elicited antibody. In some cases, a molecule in the array is a paratope or a paratope mimetic, comprising a site in the variable region of an antibody (or T cell receptor) that binds to an epitope an antigen. In some cases, an array of the disclosure is a peptide array comprising random peptide sequences.

An intra-amino acid distance in a peptide array is the distance between each peptide in a peptide microarray. An intra-amino acid distance can contribute to an off-target binding and/or to an avidity of binding of a molecule to an array. An intra-amino acid difference can be about 0.5 nm, about 1 nm, about 1 nm, 1.1 nm, about 1.2 nm, about 1.3 nm, about 1.4 nm, about 1.5 nm, about 1.6 nm, about 1.7 nm, about 1.8 nm, about 1.9 nm, about 2 nm, about 2.1 nm, about 2.2 nm, about 2.3 nm, about 2.4 nm, about 2.5 nm, about 2.6 nm, about 2.7 nm, about 2.8 nm, about 2.9 nm, about 3 nm, about 3.1 nm, about 3.2 nm, about 3.3 nm, about 3.4 nm, about 3.5 nm, about 3.6 nm, about 3.7 nm, about 3.8 nm, about 3.9 nm, about 4 nm, about 4.1 nm, about 4.2 nm, about 4.3 nm, about 4.4 nm, about 4.5 nm, about 4.6 nm, about 4.7 nm, about 4.8 nm, about 4.9 nm, about 5 nm, about 5.1 nm, about 5.2 nm, about 5.3 nm, about 5.4 nm, about 5.5 nm, about 5.6 nm, about 5.7 nm, about 5.8 nm, about 5.9 nm, and/or about 6 nm. In some cases, the intra-amino acid distance is less than 6 nanometers (nm).

An intra-amino acid difference can be at least 0.5 nm, at least 1 nm, at least 1 nm, at least 1.1 nm, at least 1.2 nm, at least 1.3 nm, at least 1.4 nm, at least 1.5 nm, at least 1.6 nm, at least 1.7 nm, at least 1.8 nm, at least 1.9 nm, at least 2 nm, at least 2.1 nm, at least 2.2 nm, at least 2.3 nm, at least 2.4 nm, at least 2.5 nm, at least 2.6 nm, at least 2.7 nm, at least 2.8 nm, at least 2.9 nm, at least 3 nm, at least 3.1 nm, at least 3.2 nm, at least 3.3 nm, at least 3.4 nm, at least 3.5 nm, at least 3.6 nm, at least 3.7 nm, at least 3.8 nm, at least 3.9 nm, at least 4 nm, at least 4.1 nm, at least 4.2 nm, at least 4.3 nm, at least 4.4 nm, at least 4.5 nm, at least 4.6 nm, at least 4.7 nm, at least 4.8 nm, at least 4.9 nm, at least 5 nm, at least 5.1 nm, at least 5.2 nm, at least 5.3 nm, at least 5.4 nm, at least 5.5 nm, at least 5.6 nm, at least 5.7 nm, at least 5.8 nm, or at least 5.9 nm.

An intra-amino acid difference can be not more than 0.5 nm, not more than 1 nm, not more than 1 nm, not more than 1.1 nm, not more than 1.2 nm, not more than 1.3 nm, not more than 1.4 nm, not more than 1.5 nm, not more than 1.6 nm, not more than 1.7 nm, not more than 1.8 nm, not more than 1.9 nm, not more than 2 nm, not more than 2.1 nm, not more than 2.2 nm, not more than 2.3 nm, not more than 2.4 nm, not more than 2.5 nm, not more than 2.6 nm, not more than 2.7 nm, not more than 2.8 nm, not more than 2.9 nm, not more than 3 nm, not more than 3.1 nm, not more than 3.2 nm, not more than 3.3 nm, not more than 3.4 nm, not more than 3.5 nm, not more than 3.6 nm, not more than 3.7 nm, not more than 3.8 nm, not more than 3.9 nm, not more than 4 nm, not more than 4.1 nm, not more than 4.2 nm, not more than 4.3 nm, not more than 4.4 nm, not more than 4.5 nm, not more than 4.6 nm, not more than 4.7 nm, not more than 4.8 nm, not more than 4.9 nm, not more than 5 nm, not more than 5.1 nm, not more than 5.2 nm, not more than 5.3 nm, not more than 5.4 nm, not more than 5.5 nm, not more than 5.6 nm, not more than 5.7 nm, not more than 5.8 nm, not more than 5.9 nm, and/or not more than 6 nm. In some cases, the intra-amino acid distance is not more than 6 nanometers (nm).

An intra-amino acid difference can range from 0.5 nm to 1 nm, 0.5 nm to 2 nm, 0.5 nm to 3 nm, 0.5 nm to 3 nm, 0.5 nm to 4 nm, 0.5 nm to 5 nm, 0.5 nm to 6 nm, 1 nm to 2 nm, 1 nm to 3 nm, 1 nm to 4 nm, 1 nm to 5 nm, 1 nm to 6 nm, 2 nm to 3 nm, 2 nm to 4 nm, 2 nm to 5 nm, 2 nm to 6 nm, 3 nm to 4 nm, 3 nm to 5 nm, 3 nm to 6 nm, 4 nm to 5 nm, 4 nm to 6 nm, and/or 5 nm to 6 nm.

A peptide array can comprise a plurality of different peptides patterns a surface. A peptide array can comprise, for example, a single, a duplicate, a triplicate, a quadruplicate, a quintuplicate, a sextuplicate, a septuplicate, an octuplicate, a nonuplicate, and/or a decuplicate replicate of the different pluralities of peptides and/or molecules. In some cases, pluralities of different peptides are spotted in replica on the surface of a peptide array. A peptide array can, for example, comprise a plurality of peptides homogenously distributed on the array. A peptide array can, for example, comprise a plurality of peptides heterogeneously distributed on the array.

A peptide can be "spotted" in a peptide array. A peptide spot can have various geometric shapes, for example, a peptide spot can be round, square, rectangular, and/or triangular. A peptide spot can have a plurality of diameters. Non-limiting examples of peptide spot diameters are about 3 µm to about 8 µm, about 3 to about 10 mm, about 5 to about 10 mm, about 10 µm to about 20 µm, about 30 µm, about 40 µm, about 50 µm, about 60 µm, about 70 µm, about 80 µm, about 90 µm, about 100 µm, about 110 µm, about 120 µm, about 130 µm, about 140 µm, about 150 µm, about 160 µm, about 170 µm, about 180 µm, about 190 µm, about 200 µm, about 210 µm, about 220 µm, about 230 µm, about 240 µm, and/or about 250 µm.

A peptide array can comprise a number of different peptides. In some cases, a peptide array comprises about 10 peptides, about 50 peptides, about 100 peptides, about 200 peptides, about 300 peptides, about 400 peptides, about 500 peptides, about 750 peptides, about 1000 peptides, about 1250 peptides, about 1500 peptides, about 1750 peptides, about 2,000 peptides; about 2,250 peptides; about 2,500 peptides; about 2,750 peptides; about 3,000 peptides; about 3,250 peptides; about 3,500 peptides; about 3,750 peptides; about 4,000 peptides; about 4,250 peptides; about 4,500 peptides; about 4,750 peptides; about 5,000 peptides; about 5,250 peptides; about 5,500 peptides; about 5,750 peptides; about 6,000 peptides; about 6,250 peptides; about 6,500 peptides; about 7,500 peptides; about 7,725 peptides 8,000 peptides; about 8,250 peptides; about 8,500 peptides; about 8,750 peptides; about 9,000 peptides; about 9,250 peptides; about 10,000 peptides; about 10,250 peptides; about 10,500 peptides; about 10,750 peptides; about 11,000 peptides; about 11,250 peptides; about 11,500 peptides; about 11,750 peptides; about 12,000 peptides; about 12,250 peptides; about 12,500 peptides; about 12,750 peptides; about 13,000 peptides; about 13,250 peptides; about 13,500 peptides; about 13,750 peptides; about 14,000 peptides; about 14,250 peptides; about 14,500 peptides; about 14,750 peptides; about 15,000 peptides; about 15,250 peptides; about 15,500 peptides; about 15,750 peptides; about 16,000 peptides; about 16,250 peptides; about 16,500 peptides; about 16,750 peptides; about 17,000 peptides; about 17,250 peptides; about 17,500 peptides; about 17,750 peptides; about 18,000 peptides; about 18,250 peptides; about 18,500 peptides; about 18,750 peptides; about 19,000 peptides; about 19,250 peptides; about 19,500 peptides; about 19,750 peptides; about 20,000 peptides; about 20,250 peptides; about 20,500 peptides; about 20,750 peptides; about 21,000 peptides; about 21,250 peptides; about 21,500 peptides; about 21,750 peptides; about 22,000 peptides; about 22,250 peptides; about 22,500 peptides; about 22,750 peptides; about 23,000 peptides; about 23,250 peptides; about 23,500 peptides; about 23,750 peptides; about 24,000 peptides; about 24,250 peptides; about 24,500 peptides; about 24,750 peptides; about 25,000 peptides; about 25,250 peptides; about 25,500 peptides; about 25,750 peptides; and/or about 30,000 peptides.

In some cases, a peptide array used in a method of health monitoring, a method of treatment, a method of diagnosis, and a method for preventing a condition comprises more than 30,000 peptides. In some cases, a peptide array used in a method of health monitoring comprises about 330,000 peptides. In some cases the array comprise about 30,000 peptides; about 35,000 peptides; about 40,000 peptides; about 45,000 peptides; about 50,000 peptides; about 55,000 peptides; about 60,000 peptides; about 65,000 peptides; about 70,000 peptides; about 75,000 peptides; about 80,000 peptides; about 85,000 peptides; about 90,000 peptides; about 95,000 peptides; about 100,000 peptides; about 105,000 peptides; about 110,000 peptides; about 115,000 peptides; about 120,000 peptides; about 125,000 peptides; about 130,000 peptides; about 135,000 peptides; about 140,000 peptides; about 145,000 peptides; about 150,000 peptides; about 155,000 peptides; about 160,000 peptides; about 165,000 peptides; about 170,000 peptides; about 175,000 peptides; about 180,000 peptides; about 185,000 peptides; about 190,000 peptides; about 195,000 peptides; about 200,000 peptides; about 210,000 peptides; about 215,000 peptides; about 220,000 peptides; about 225,000 peptides; about 230,000 peptides; about 240,000 peptides; about 245,000 peptides; about 250,000 peptides; about 255,000 peptides; about 260,000 peptides; about 265,000 peptides; about 270,000 peptides; about 275,000 peptides; about 280,000 peptides; about 285,000 peptides; about 290,000 peptides; about 295,000 peptides; about 300,000 peptides; about 305,000 peptides; about 310,000 peptides; about 315,000peptides; about 320,000 peptides; about 325,000 peptides; about 330,000 peptides; about 335,000 peptides; about 340,000 peptides; about 345,000 peptides; and/or about 350,000 peptides. In some cases, a peptide array used in a method of health monitoring comprises more than 330,000 peptides.

A peptide array can comprise a number of different peptides. In some cases, a peptide array comprises at least 2,000 peptides; at least 3,000 peptides; at least 4,000 peptides; at least 5,000 peptides; at least 6,000 peptides; at least 7,000 peptides; at least 8,000 peptides; at least 9,000 peptides; at least 10,000 peptides; at least 11,000 peptides; at least 12,000 peptides; at least 13,000 peptides; at least 14,000 peptides; at least 15,000 peptides; at least 16,000 peptides; at least 17,000 peptides; at least 18,000 peptides; at least 19,000 peptides; at least 20,000 peptides; at least 21,000 peptides; at least 22,000 peptides; at least 23,000 peptides; at least 24,000 peptides; at least 25,000 peptides; at least 30,000 peptides; at least 40,000 peptides; at least 50,000 peptides; at least 60,000 peptides; at least 70,000 peptides; at least 80,000 peptides; at least 90,000 peptides; at least 100,000 peptides; at least 110,000 peptides; at least 120,000 peptides; at least 130,000 peptides; at least 140,000 peptides; at least 150,000 peptides; at least 160,000 peptides; at least about 170,000 at least 180,000 peptides; at least 190,000 peptides; at least 200,000 peptides; at least 210,000 peptides; at least 220,000 peptides; at least 230,000 peptides; at least 240,000 peptides; at least 250,000 peptides; at least 260,000 peptides; at least 270,000 peptides; at least 280,000 peptides; at least 290,000 peptides; at least 300,000 peptides; at least 310,000 peptides; at least 320,000 peptides; at least 330,000 peptides; at least 340,000 peptides; at least 350,000 peptides. In some cases, a peptide array used in a method of health monitoring comprises at least 330,000 peptides.

A peptide can be physically tethered to a peptide array by a linker molecule. The N- or the C-terminus of the peptide can be attached to a linker molecule. A linker molecule can be, for example, a functional plurality or molecule present on the surface of an array, such as an imide functional group, an amine functional group, a hydroxyl functional group, a carboxyl functional group, an aldehyde functional group, and/or a sulfhydryl functional group. A linker molecule can be, for example, a polymer. In some cases the linker is maleimide. In some cases the linker is a glycine-serine-cysteine (GSC) or glycine-glycine-cysteine (GGC) linker. In some cases, the linker consists of a polypeptide of various lengths or compositions. In some cases the linker is polyethylene glycol of different lengths. In yet other cases, the linker is hydroxymethyl benzoic acid, 4-hydroxy-2-methoxy benzaldehyde, 4-sulfamoyl benzoic acid, or other suitable for attaching a peptide to the solid substrate.

A surface of a peptide array can comprise a plurality of different materials. A surface of a peptide array can be, for example, glass. Non-limiting examples of materials that can comprise a surface of a peptide array include glass, functionalized glass, silicon, germanium, gallium arsenide, gallium phosphide, silicon dioxide, sodium oxide, silicon nitrade, nitrocellulose, nylon, polytetraflouroethylene, polyvinylidendiflouride, polystyrene, polycarbonate, methacrylates, or combinations thereof.

A surface of a peptide array can be flat, concave, or convex. A surface of a peptide array can be homogeneous and a surface of an array can be heterogeneous. In some cases, the surface of a peptide array is flat.

A surface of a peptide array can be coated with a coating. A coating can, for example, improve the adhesion capacity of an array of the invention. A coating can, for example, reduce background adhesion of a biological sample to an array of the invention. In some cases, a peptide array of the invention comprises a glass slide with an aminosilane-coating.

A peptide array can have a plurality of dimensions. A peptide array can be a peptide microarray.

### Detection.

Binding interactions between components of a sample and an array can be detected in a variety of formats. In some formats, components of the samples are labeled. The label can be a radioisotype or dye among others. The label can be supplied either by administering the label to a patient before obtaining a sample or by linking the label to the sample or selective component(s) thereof.

Binding interactions can also be detected using a secondary detection reagent, such as an antibody. For example, binding of antibodies in a sample to an array can be detected using a secondary antibody specific for the isotype of an antibody (e.g., IgG (including any of the subtypes, such as IgG1, IgG2, IgG3 and IgG4), IgA, IgM). The secondary antibody is usually labeled and can bind to all antibodies in the sample being analyzed of a particular isotype. Different secondary antibodies can be used having different isotype specificities. Although there is often substantial overlap in compounds bound by antibodies of different isotypes in the same sample, there are also differences in profile.

Binding interactions can also be detected using label-free methods, such as surface plasmon resonance (SPR) and mass spectrometry. SPR can provide a measure of dissociation constants, and dissociation rates. The A-100 Biocore/GE instrument, for example, is suitable for this type of analysis. FLEXchips can be used to analyze up to 400 binding reactions on the same support.

Optionally, binding interactions between component(s) of a sample and the array can be detected in a competition format. A difference in the binding profile of an array to a sample in the presence versus absence of a competitive inhibitor of binding can be useful in characterizing the sample. The competitive inhibitor can be for example, a known protein associated with a disease condition, such as pathogen or antibody to a pathogen. A reduction in binding of member(s) of the array to a sample in the presence of such a competitor provides an indication that the pathogen is present. The stringency can be adjusted by varying the salts, ionic strength, organic solvent content and temperature at which library members are contacted with the target.

An antibody based method of detection, such as an enzyme-linked immunosorbent assay (ELISA) method can be used to detect a pattern of binding to an array of the invention. For example, a secondary antibody that detects a particular isotype of an immunoglobulin, for example the IgM isotype, can be used to detect a binding pattern of a plurality of IgM antibodies from a complex biological sample of a subject to an array. The secondary antibody can be, for example conjugated to a detectable label, such as a fluorescent moiety or a radioactive label.

The disclosure provides arrays and methods for the detection of an off-target binding of a plurality of different antibodies to an array of the disclosure. A plurality of antibodies in a complex biological sample are capable of off-target binding of a plurality of peptides in a peptide microarray. In some cases, detecting an off-target binding of at least one antibody to a plurality of peptides in the peptide array can form an immunosignature. In other cases detecting an off-target binding of at least one antibody to at least 10 different peptides in the peptide array can form an immunosignature. In yet other cases detecting an off-target binding of at least one antibody to at least 20 different peptides in the peptide array can form an immunosignature. In still other cases detecting an off-target binding of at least one antibody to at least 50 different peptides in the peptide array can form an immunosignature. In yet other cases detecting an off-target binding of at least one antibody to at least 100 different peptides in the peptide array can form an immunosignature. A plurality of classes or isotypes of antibodies can provide an off-target pattern of binding to an array. An antibody, or immunoglobulin, can be an IgA, IgD, IgE, IgG, and/or an IgM antibody.

A pattern of binding of at least one IgM antibody from a complex biological sample to a peptide array can form an immunosignature. An IgM antibody can form polymers where multiple immunoglobulins are covalently linked together with disulfide bonds. An IgM polymer can be a pentamer. The polymeric nature of an antibody with the IgM isotype can increase off-target binding of a sample to an array. A polymeric nature of an antibody can increase an avidity of binding of a sample to an array. For example, a pattern of binding of antibodies of a polymeric IgM isotype antibodies to a peptide microarray can form a unique pentameric driven immunosignature.

An IgA antibody can be an IgA1 or an IgA2 antibody. An antibody of the IgA isotype can form a dimer. An IgG antibody can be an IgG1, IgG2, IgG3, or an IgG4 antibody. An antibody of the IgG isotype can exist as a monomer. An IgD and/or an IgE antibody can form a monomer. In some cases, the disclosure can detect an off-target binding of at least one IgM antibody from a complex biological sample of a subject to a peptide array.

### Treatments and Conditions.

The methods and arrays of the disclosure provide sensitive methods for the identification of a therapeutic target, and/or a vaccine. The methods and arrays of the disclosure can be used for a screening of therapeutic targets against a cancer and/or immune disorder of conditions. The array and methods of the disclosure can be used, for example, to identify a therapeutic target of a plurality of different conditions of a subject. A subject can be a human, a guinea pig, a dog, a cat, a horse, a mouse, a rabbit, and various other animals. A subject can be of any age, for example, a subject can be an infant, a toddler, a child, a pre-adolescent, an adolescent, an adult, or an elderly individual.

A condition of a subject can correspond to a disease or a healthy condition. In some cases, a condition of a subject is a healthy condition, and a method of the disclosure monitors the healthy condition. In some cases, a condition of a subject is a disease condition, and a method of the disclosure is used to diagnose/monitor a state and/or the progression of the condition. A method of the disclosure can also be used in the prevention of a condition. In some cases, a method of the disclosure is used in conjunction with a prophylactic treatment.

In some cases, a method of the disclosure is a method for identifying a therapeutic target against an autoimmune disorder, the method comprising: a) contacting a peptide array with a first biological sample from an individual with a known autoimmune disorder of interest; b) detecting binding of antibodies in the first biological sample with the peptide array to obtain a first immunosignature profile; c) contacting a peptide array with a control sample derived from an individual without the known autoimmune disorder; d) detecting binding of antibody in the control sample with the peptide array to obtain a second immunosignature profile; e) comparing the first immunosignature profile to the second immunosignature profile and identifying differentially bound peptides that either bind less or more antibody in the first immunosignature profile as compared to the second immunosignature profile; and f) identifying proteins that correspond to the identified differentially bound peptides as targets against the autoimmune disorder of interest.

In some cases, a method of the disclosure can be used to for example, of identifying a therapeutic target, vaccine, or screening for therapeutic targets against an immune disorder. Non-limiting examples of disorders associated with the immune system can include: auto-immune disorders, inflammatory diseases, HIV, rheumatoid arthritis, diabetes mellitus type 1, systemic lupus erythematosus, scleroderma, multiple sclerosis, severe combined immunodeficiency (SCID), DiGeorge syndrome, ataxia-telangiectasia, seasonal allergies, perennial allergies, food allergies, anaphylaxis, mastocytosis, allergic rhinitis, atopic dermatitis, Parkinson's, Alzheimer's, hypersplenism, leukocyte adhesion deficiency, X-linked lymphoproliferative disease, X-linked agammaglobulinemia, selective immunoglobulin A deficiency, hyper IgM syndrome, autoimmune lymphoproliferative syndrome, Wiskott-Aldrich syndrome, chronic granulomatous disease, common variable immunodeficiency (CVID), hyperimmunoglobulin E syndrome, and Hashimoto's thyroiditis.

In some embodiments, the immune disorder is an auto-immune disorder. In some embodiments the auto-immune disorder is chosen from the group consisting of Type I diabetes, rheumatoid arthritis, multiple sclerosis, inflammatory bowel disease, systemic lupus erythematosus, psoriasis, and scleroderma.

The disclosure can provide a method of preventing a condition, the method comprising: a) providing a complex biological sample from a subject; b) contacting the complex biological sample to a peptide array, wherein the peptide array comprises different peptides capable of off-target binding of at least one antibody in the complex biological sample; c) measuring an off-target binding of the complex biological sample to a plurality of the different peptides to form an immunosignature; d) associating the immunosignature with a condition; and e) receiving a treatment for the condition.

In some cases, a method of the disclosure can be used in conjunction with a prophylactic treatment. Vaccines, for example, can be prophylactic treatments. Non-limiting examples of vaccines that function as prophylactic treatments include polio vaccines, smallpox vaccines, measles vaccines, mumps vaccines, human papillomavirus (HPV) vaccines, and influenza vaccines. In some cases, a method of the disclosure is used to monitor, for example, a subject's response to a prophylactic vaccine.

In some cases, the disclosure provides a method of identifying vaccine targets comprising: a. contacting a peptide array with a first biological sample from an individual with a known condition of interest; b. detecting binding of antibodies in the first biological sample with the peptide array to obtain a first immunosignature profile; c. contacting a peptide array with a control sample derived from an individual without the known condition; d. detecting binding of antibody in the control sample with the peptide array to obtain a second immunosignature profile; e. comparing the first immunosignature profile to the second immunosignature profile and identifying differentially bound peptides that either bind less or more antibody in the first immunosignature profile as compared to the second immunosignature profile; and f. identifying proteins that correspond to the identified differentially bound peptides as vaccine targets for the condition of interest.

In some cases, the vaccine is against a pathogen, a microbial organism, a virus, a cancer or an autoimmune disorder. A pathogen can be a pathogenic virus or a pathogenic bacteria. An infection with a pathogenic viruses and/or a pathogenic bacteria can cause a condition, for example, an inflammation. Non-limiting examples of pathogenic bacteria can be found in the: a) Bordetella genus, such as *Bordetella pertussis* species; b) Borrelia genus, such *Borrelia burgdorferi* species; c) Brucelia genus, such as *Brucella abortus, Brucella canis, Brucela meliterisis,* and/or *Brucella suis* species; d) Campylobacter genus, such as *Campylobacter jejuni* species; e) Chlamydia and Chlamydophila genuses, such as *Chlamydia pneumonia, Chlamydia trachomatis,* and/or *Chlamydophila psittaci* species; f) Clostridium genus, such as *Clostridium botulinum, Clostridium difficile, Clostridium perfringens, Clostridium tetani* species; g) Corynebacterium genus, such as *Corynebacterium diphtheria* species; h) Enterococcus genus, such as *Enterococcus faecalis,* and/or *Enterococcus faecium* species; i) Escherichia genus, such as *Escherichia coli* species; j) Francisella genus, such as *Francisella tularensis* species; k) Haemophilus genus, such as *Haemophilus influenza* species; l) Helicobacter genus, such as *Helicobacter pylori* species; m) Legionella genus, such as *Legionella pneumophila* species; n) Leptospira genus, such as *Leptospira interrogans* species; o) Listeria genus, such as *Listeria monocytogenes* species; p) Mycobacterium genus, such as *Mycobacterium leprae, mycobacterium tuberculosis,* and/or *mycobacterium ulcerans* species; q) Mycoplasma genus, such as *Mycoplasma pneumonia* species; r) Neisseria genus, such as *Neisseria gonorrhoeae* and/or *Neisseria meningitidia* species; s) Pseudomonas genus, such as *Pseudomonas aeruginosa* species; t) Rickettsia genus, such as *Rickettsia rickettsii* species; u) Salmonella genus, such as *Salmonella typhi* and/or *Salmonella typhimurium* species; v) Shigella genus, such as *Shigella sonnei* species; w) Staphylococcus genus, such as *Staphylococcus aureus, Staphylococcus epidermidis,* and/or *Staphylococcus saprophyticus* species; x) Streptpcoccus genus, such as *Streptococcus agalactiae, Streptococcus pneumonia,* and/or *Streptococcus pyogenes* species; y) Treponema genus, such as *Treponema pallidum* species; z) Vibrio genus, such as *Vibrio cholera;* and/or aa) Yersinia genus, such as *Yersinia pestis* species.

Non-limiting examples of viruses can be found in the following families of viruses and are illustrated with exemplary species: a) Adenoviridae family, such as Adenovirus species; b) Herpesviridae family, such as Herpes simplex type 1, Herpes simplex type 2, Varicella-zoster virus, Epstein-barr virus, Human cytomegalovirus, Human herpesvirus type 8 species; c) Papillomaviridae family, such as Human papillomavirus species; d) Polyomaviridae family, such as BK virus, JC virus species; e) Poxviridae family, such as Smallpox species; f) Hepadnaviridae family, such as Hepatitis B virus species; g) Parvoviridae family, such as Human bocavirus, Parvovirus B19 species; h) Astroviridae family, such as Human astrovirus species; i) Caliciviridae family, such as Norwalk virus species; j) Flaviviridae family, such as Hepatitis C virus, yellow fever virus, dengue virus, West Nile virus species; k) Togaviridae family, such as Rubella virus species; 1) Hepeviridae family, such as Hepatitis E virus species; m) Retroviridae family, such as Human immunodeficiency virus (HIV) species; n) Orthomyxoviridaw family, such as Influenza virus species; o) Arenaviridae family, such as Guanarito virus, Junin virus, Lassa virus, Machupo virus, and/or Sabiá virus species; p) Bunyaviridae family, such as Crimean-Congo hemorrhagic fever virus species; q) Filoviridae family, such as Ebola virus and/or Marburg virus species; Paramyxoviridae family, such as Measles virus, Mumps virus, Parainfluenza virus, Respiratory syncytial virus, Human metapneumovirus, Hendra virus and/or Nipah virus species; r) Rhabdoviridae genus, such as Rabies virus species; s) Reoviridae family, such as Rotavirus, Orbivirus, Coltivirus and/or Banna virus species. In some cases, a virus is unassigned to a viral family, such as Hepatitis D.

In some embodiments, the vaccine is against a microbial organism. A microbial organism can be a single cell or a multicellular organism. A microbial organism can be a bacteria, a virus, a fungi, or a protozoon.

The array devices and methods disclosed herein provide sensitive methods for the identification of a therapeutic target, and/or a vaccine from a variety of diseases and/or conditions simultaneously. The methods and arrays of the invention also provide sensitive methods for a screening of therapeutic targets against a cancer and/or immune disorder of conditions from a variety of diseases and/or conditions simultaneously. For example, the array devices and methods disclosed herein are capable of simultaneously detecting inflammatory conditions, cancer diseases and pathogenic infection on the same array. Accordingly, only one array, *i.e.* one immunosignature assay, is necessary to detect a wide spectra of diseases and conditions. Thus, the monitoring of a subject through its lifespan will provide, with every immunosignature performed, a snapshot through time of the subject's health status. This provides a powerful means of detecting global and specific changes in the subject's health status, and together with the high sensitivity of the immunosignature assay, provides a system capable of detecting at very early stages any change in the individual's health status.

Accordingly, the methods, systems and array devices disclosed herein are capable of screening, identifying therapeutic targets, identifying vaccine targets, and/or treating a disease and/or condition at an early stage of the disease and/or condition. For example, the methods, systems and array devices disclosed herein are capable of detecting, diagnosing and monitoring a disease and/or condition days or weeks before traditional biomarker-based assays. Moreover, only one array, *i.e.,* one immunosignature assay, is needed to detect, diagnose and monitor a side spectra of diseases and conditions, including inflammatory conditions, cancer and pathogenic infections.

### Classification Algorithms.

A plurality of algorithms and classifiers can be used to classify and/or analyze data obtained in an Immunosignaturing array. The Naive Bayes' algorithm can accommodate the complex patterns hidden within multilayered immunosignaturing microarray data due to its fundamental mathematical properties. A basic classification algorithm, Linear Discriminant Analysis (LDA) is widely used in analyzing biomedical data in order to classify two or more disease classes. LDA can be, for example, a classification algorithm. A comparative study of common classifiers described in the art is describe in (Kukreja et al, BMC Bioinformatics. 2012; 13: 139).

### EXAMPLES

### EXAMPLE 1:

The following examples describe exemplary systems and methods for identifying peptide(s) in a peptide array.

### Deciphering the Identity of Peptides in a Type I Diabetes Immunosignature.

Example 1 illustrates methods and systems applied to the identification of a Type I Diabetes Immunosignature. Sera from 40 Type I diabetic subjects and 40 age matched controls was applied to a random peptide microarray comprising ∼10,000 peptides. A pattern of binding of the antibodies in the sera was detected and quantified. A t-test was used to select peptides that met the threshold of at least p<0.0001 false discovery rate. At this false discovery rate, 689 differential peptides were identified amongst the 40 Type I diabetes and 40 age matched controls. **TABLE 1** describes the performance measures of different peptides in Immuosignaturing of Type I diabetes, and the classification accuracy, specificity and sensitivity for these differential peptides.

**TABLE 1**

| **TABLE 1** | |
|---|---|
| | Type I Diabetes Vs Controls (IgG) |
| No. of Features | 689 (p <0.0001) ^{a} |
| Accuracy | 88.75^{b} |
| Specificity | 92.3 ^{b} |
| Sensitivity | 85.4^{b} |

| | |
|---|---|
| legend: ^{a} Denotes number of peptides selected from 10,000 (2-tail) from Welsh t-test with overall Type I error less than 0.0001 that are differential in 2 conditions. ^{b} Denotes performance measure obtained on leave-one-out cross validation by Naive Bayes classification algorithm on 40 Type I diabetes and 40 controls subjects. | |

We were able to achieve >90% specificity using Naive Bayes classification algorithm leave-one-out-cross-validation though Immunosignaturing. Leave-one-out cross validation is a method of eliminating classification accuracy in which one individual is excluded from the set of samples. The algorithm is trained on the remaining set and used to predict the left out sample. All samples are tested in turn and the percent accuracy calculated. The distribution of the 679 differential peptides was skewed with only 210 peptides up regulated and 479 peptides down regulated compared to controls. We then asked as to how many peptides can be bioinformatically mapped to the known antigens in Type I diabetes.

### Decipher of Type I Diabetes Immunosignature.

We first considered 210 up regulated peptides in Type I diabetes, and bioinformatically mapped it to 8 known Type I diabetes antigens using the epitope matching tool GUITOPE. The protein sequence for 8 known antigens was matched against homology with 210 peptides. Peptides showing significant matching on a region in protein (epitope) over equal number of peptides from our 10,000 peptide library at p<0.0001 were selected.

On an average, we were able to successfully map peptides from 210 to known antigens with 1-2 epitopes per antigen. 3-4 peptides on an average mapped with one epitope of an antigen. 7 peptides on an average are mapped against antigen from up regulated set of peptides. **TABLE 2** corresponds to the mapping of 210 up regulated peptides from immunosignature to 8 known antigens in Type I diabetes. **TABLE 2** shows the number of epitopes, peptides per epitope and total peptides mapped per antigen for up regulated peptides 210 peptides over 8 known antigens in Type I diabetes.

**TABLE 2**

| **TABLE 2** | | | | | |
|---|---|---|---|---|---|
| **Protein** ^{a} **Upreg.** | # **Epitopes** ^{b} | # **Peptides-E1** ^{c} | # **Peptides-E2** ^{c} | **Total** ^{d} | **FDR**^{e} |
| ICA-69 | 2 | 5 | 3 | 8 | <0.0001 |
| GLUT-2 | 2 | 3 | 3 | 6 | <0.0001 |
| GAD-67 | 2 | 4 | 3 | 7 | <0.0001 |
| GAD-65 | 1 | 13 | NA | 13 | <0.0001 |
| IA-2 β | 2 | 3 | 4 | 7 | <0.0001 |
| Insulin | 2 | 3 | 4 | 7 | <0.0001 |
| ZnT8 | 2 | 4 | 3 | 7 | <0.0001 |
| IA-2 | 1 | 1 | NA | 1 | <0.0001 |

| | | | | | |
|---|---|---|---|---|---|
| legend: **^{a}** Denotes self protein/antigens reported in Type I diabetes. **^{b}** Denotes total number of epitopes observed bioinformatically using GUITOPE on a protein. **^{c}** Denotes no. of peptides that mapped against a particular epitope E1/E2 of a protein. **^{d}** Denotes total number of peptides from 210 up regulated peptides in Type I diabetes that mapped to an antigen. **^{e}**Denotes False Discovery rate, probability of observing equivalent matching against an antigen when equal number of peptides are chosen from 10,000 random peptide library. | | | | | |

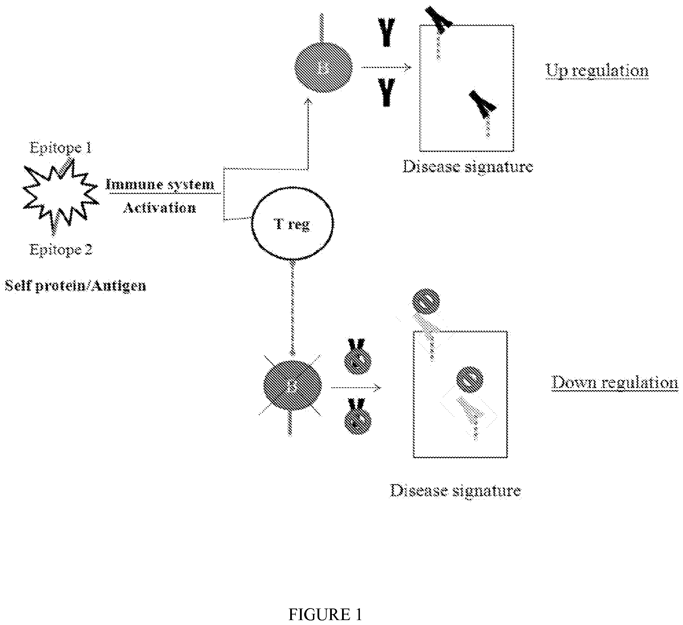

Next, we considered the remaining 479 down regulated peptides and mapped them against the known 8 antigens in Type 1 diabetes. Our hypothesis behind this is presented in **FIGURE 1. FIGURE 1** is a schematic of a pathway showing how a self protein/antigen can lead to up-regualtion and down-regulation of Immunosignaturing in random sequence peptide array. **FIGURE 1** illustrates the concept that disease associated proteins contain multiple epitopes, by illustrating a self-protein with two epitopes. In **FIGURE 1****,** the self protein containing two epitopes is encountered by antigen presenting cells and activates B cells. Epitope 1 results in the activation of a B cell which begins producing antibodies reactive to Epitope 1, and these antibodies are then detected on the peptide array through an increase in signal on antibody reactive peptides. Conversely when Epitope 2 activates a B cell there is an epitope specific regulatory T cell (Treg), that suppresses antibody secretion by the B cell. As a result antibodies are not replaced as they are depleted from circulation leading to down regulation or lack of binding of these antibodies to an array of the invention.

**TABLE 3** shows the 479 down regulated peptides from immunosignature mapped to 8 known antigens in Type I diabetes. Among down regulated peptides, peptides were mapped against only GAD-65, ZnT8 and Insulin, which are the strong antigens in Type I diabetes. The number of mapped peptides in down regulation were higher compared to up regulated set of peptides but the number of epitopes per antigen were similar for both up regulation and down regulation. Peptides mapped to the antigens for two cases are indeed not only different since they are coming from two distinct sets, but also they mapped epitopes are different on the protein sequence.

Hence a particular epitope of an antigen can result in up regulation of peptides while other epitope on the same protein can result in down regulation of some other set of peptides. **TABLE 3.** Mapping of 479 down regulated peptides from Immunosignature to 8 known antigens in Type I diabetes.

**TABLE 3**

| **TABLE 3** | | | | | |
|---|---|---|---|---|---|
| **Protein** ^{a} **Downreg** | # **Epitopes** ^{b} | # **Peptides-E1** ^{c} | #**Peptides-E2** ^{c} | **Total** ^{d} | **FDR** ^{e} |
| ICA-69 | - | - | - | - | >0.999 |
| GLUT-2 | - | - | - | - | >0.999 |
| GAD-67 | - | - | - | - | >0.999 |
| GAD-65 | 2 | 25 | 17 | 42 | <0.0001 |
| IA-2 0 | - | - | - | - | >0.999 |
| Insulin | 2 | 9 | 14 | 23 | <0.0001 |
| ZnT8 | 1 | 16 | - | 16 | <0.0001 |
| IA-2 | - | - | - | - | >0.999 |

| | | | | | |
|---|---|---|---|---|---|
| legend: **^{a}** Denotes self protein/antigens reported in Type I diabetes. **^{b}** Denotes total number of epitopes observed bioinformatically using GUITOPE on a protein. **^{c}** Denotes no. of peptides that mapped against a particular epitope E1/E2 of a protein. **^{d}** Denotes total number of peptides from 479 down regulated peptides in type 1 diabetes that mapped to an antigen. **^{e}** Denotes False Discovery rate, probability of observing equivalent matching against an antigen when equal number of peptides are chosen from 10,000 random peptide library. | | | | | |

In order to set up a null control and method verification, we mapped differential (both up regulated and down regulated) against 8 random proteins, some related to enzymes and the rest random which are not involved in antibody production or Type I diabetes pathway.

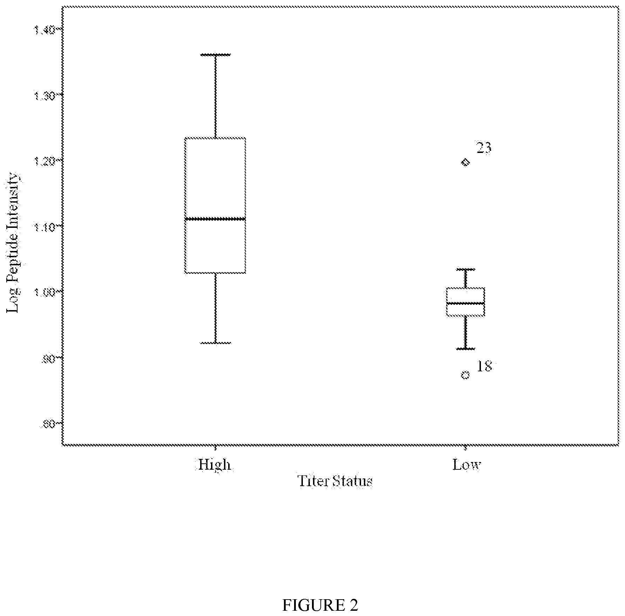

In order to provide validation and to verify the mapped peptides against known antigens, an orthogonal measurement was performed. **FIGURE 2** is a box plot showing the intensity of 13 peptides by 2 groups of Type I diabetes patients as possessing either high and low GAD-65 radio-immuno precipitation (RIP) titers. Individual T1D patients were run in an immunosignaturing assay on the CIM1OK array. Bioinformatic selection resulted in the identification of 13 peptides which mapped back to GAD-65. The individual patient serum reactivity for the 13 peptides was averaged and group values presented in a box plot. Groups were defined based on either a high or low anti-titer GAD-65. There was a significant difference between the mean of the two groups (p <0.001). **FIGURE 2** shows that no peptides were mapped against a set of 8 random proteins.

**TABLE 4** Mapping of 679 differential peptides from Immunosignature of Type I diabetes to 8 random Protein/antigens.

**TABLE 4**

| **TABLE 4** | | | |
|---|---|---|---|
| **Protein** ^{a} | # **Epitopes** ^{b} | **Total** ^{c} | **FDR** ^{d} |
| AOX | - | - | >0.99 |
| ACAA1 | - | - | >0.999 |
| ACSBG1 | - | - | >0.999 |
| ACOT1 | - | - | >0.999 |
| CPT 1 A | - | - | >0.999 |
| ALDH2 | - | - | >0.99 |
| CoA | - | - | >0.99 |
| ACLS1 | - | - | >0.99 |

| | | | |
|---|---|---|---|
| legend: **^{a}** Denotes random protein/self antigens as a part of negative control. **^{b}** Denotes total number of epitopes observed bioinformatically using GUITOPE on a protein. **^{c}** Denotes total number of peptides from all 689 differential peptides (both up regulated and down regulated) in Type I diabetes. **^{d}** Denotes False discovery rate of observing equivalent matching against same antigen when equal number of peptides are chosen from 10,000 random peptide library. | | | |

### Orthogonal measurement and validation of results.

We measured GAD-65 titers using radio-immuno-precipitation (RIP) assay and separated 40 Type I samples into two classes High/ Low GAD-65 titers and asked if there is any correspondence between 13 mapped peptides intensities against GAD-65 and RIP titers. **FIGURE 2** showed a box plot of average of 13 peptides intensities on high and low titer GAD-65, demonstrating a significant difference between the mean of the two groups (p <0.001). We then asked if there is any linear correspondence between GAD-65 R.I.P titers and the corresponding mean of 13 peptide intensities.

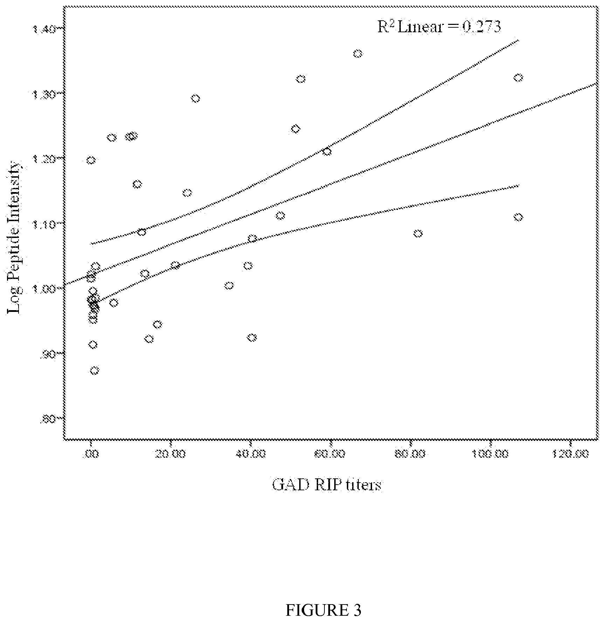

**FIGURE 3** shows a linear regression analysis of the relationship between peptide intensity on the Immunosignature array and the anti-GAD titers. The scatter plot with linear regression line in **FIGURE 3** shows a 95% confidence band of GAD-65 RIP titers and log peptide intensities. The slope of the regression line indicated a linear relationship. The mapped peptides of GAD-65 showed a linear correspondence with the RIP titer.

### Decipher of a Murine Breast Cancer Model Immunosignature.

A pressing need exists for the identification of novel treatments and vaccine candidates for Breast cancer. This example describes the application of methods and systems of the disclosure to the characterization of her2/neuT transgenic murine breast cancer model. Immunosignaturing was applied in a comparative analysis of wild-type (healthy mice) versus mice which are prone to develop breast cancer (her2/neuT mice).

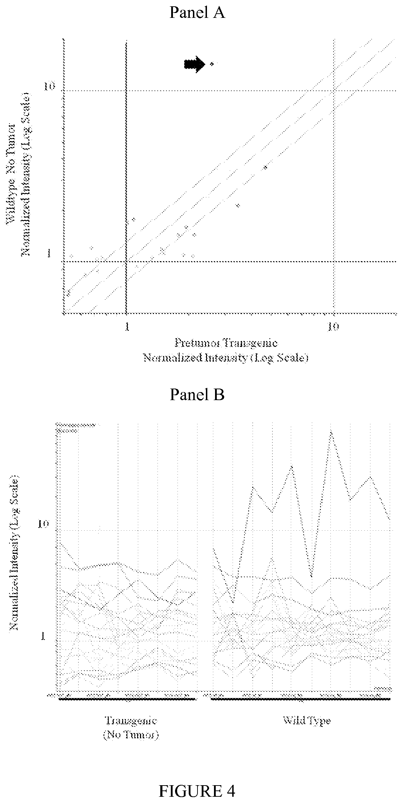

**FIGURE 4** illustrates the deciphering/identification of KSFHGRVIQDVVGEPYGGSC (SEQ ID NO. 1) as a peptide of interest. The immunosignature of age matched wildtype BALB/c mice 10 (n=8) was compared to transgenic BALB/c-neuT mice (n=11) on a CIMlOk exemplary array. Mice were 10, 15 or 16 weeks of age. The transgenic BALB/c-neuT mice did not have palpable tumors at the time of blood collection. Twenty-tree peptides were selected using a T-test p-value of less than 0.005. These 23 peptides were capable of classifying the individual mice using a Naive Bayes classification algorithm giving a leave-one-out cross-validation of 89% correct. Twenty-three peptides were identified as significant p<0.005 between groups (**FIGURE 4****,** Panel A) and capable of discriminating individual mice based on disease status using leave-one-out cross-validation in a Naive Bayes model. One of these peptides was strongly upregulated in all but one wild-type over transgenic mice (**FIGURE 4****,** Panel B). **FIGURE 4****,** panel A is a scatterplot illustrating the relationship of the peptides between the bioinformatic averages of the normalized intensities for wildtype vs the transgenic mice. The arrow in **FIGURE 4****,** Panel A, points to the peptide that is significantly up in the wildtype compared to the transgenic mice (p=2.33x10⁻⁵).

**FIGURE 4****,** panel B illustrates the normalized intensities for individual mice plotted as a line graph, where lines represent the individual peptides and the grey vertical graph lines represent individual mice. The KSFHGRVIQDVVGEPYGGSC (SEQ ID NO. 1) peptide is plotted as the black line and is constantly up in all but one mouse.

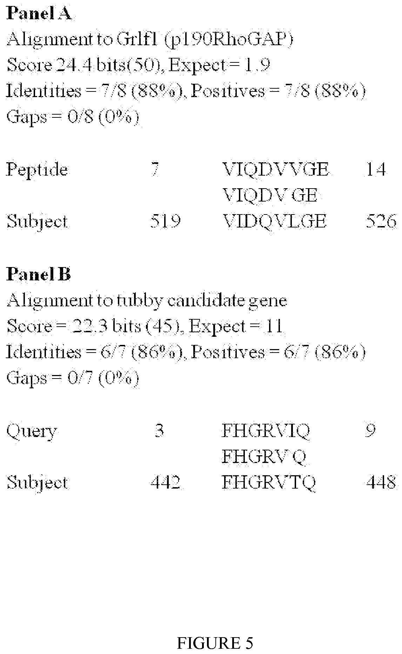

This peptide was mapped back to two putative targets, p190RhoGAP and the tubby candidate gene. **FIGURE 5** illustrates BLAST alignments of peptide KSFHGRVIQDVVGEPYGGSC (SEQ ID NO. 1) to the top two mouse proteome candidates. **FIGURE 5****,** Panel A is a BLAST alignment of the top likely match, namely p190RhoGAP. **FIGURE 5**, Panel B is a BLAST alignment of the top second likely match is tubby candidate gene. The identified peptide sequences were used to search the translated *Mus musculus* genome, build 37.2, for matches using the NCBI Basic Local Alignment Search Tool accessed online 28 OCT 2011.

The her2 gene inserted into the transgenic mice conducts signaling through p190RhoGAP and tubby has previously been associated with cancer associated retinopathy.

### Deciphering the Immunosignature of an Influenza A/PR/8/34 Infection and of a KLH Immunization of Mice.

To demonstrate the ability of the decipher approach to identify immunogenic targets during an immunization, experiments characterizing an immunosignature from BALB/c mice that were either infected with Influenza (H1N1) A/PR/8/34 or immunized with KLH were performed. Mice immunized with KLH were given 100 ug KLH in alum on days 0, 14 and 28. Infected mice were given a sublethal intranasal dose of 1 x 10⁴ viral particles. Mice from both groups were bled on day 35 and sera run on an exemplary array of the disclosure, in this case, the CIM10K array. Analysis using the GUlTOPE program revealed one epitope of interest in KLH immunized mice (**TABLE 5**). **TABLE 5** tabulates the results of the GUITOPE analysis mapping 305 differential peptides from immunosignaturing of KLH immunized mice versus PBS (Mock) immunized mice at p < 0.005.

**TABLE 5**

| **TABLE 5** | | | | | |
|---|---|---|---|---|---|
| Protein ^{a} | Regulation ^{b} | # Epitopes ^{c} | # Peptides-El ^{d} | Total ^{e} | FDR^{e} |
| KLH | Up regulated [189] | 1 | 6 | 0 | <0.0001 |
| KLH | Down-regulated [116] | 0 | 0 | 0 | >0.99 |

| | | | | | |
|---|---|---|---|---|---|
| legend: **^{a}** Denotes KLH immunization in 3 mice. **^{b}** Denotes number of peptides that were up-regulated and down regulated compared to PBS immunized mice (Mock). **^{c}** Denotes total number of epitopes observed bioinformatically using GUITOPE on a protein. **^{d}** Denotes number of peptides mapped to first epitope, **^{e}** Denotes total number of mapped peptides from either up regulated and down regulated in KLH immunized mice. **^{f}** Denotes False discovery rate of observing equivalent matching against same antigen when equal number of peptides are chosen from 10,000 random peptide library. | | | | | |

Analysis using the GUlTOPE program revealed one increasingly recognized epitope and one decreasingly recognized epitope in influenza infected mice (**TABLE 6**). **TABLE 6** tabulates the results of the GUITOPE analysis mapping 325 differential peptide from immunosignaturing of PR8 immunized mice versus PBS (Mock) immunized mice at p<0.01.

**TABLE 6**

| **TABLE 6** | | | | | |
|---|---|---|---|---|---|
| Protein^{a} | Regulation^{b} | #Epitopes ^{c} | #Peptides-El^{d} | Total ^{e} | FDR^{f} |
| PR8 | Up regulated [228] | 1 | 8 | 8 | <0.0001 |
| PR8 | Down-regulated [97] | 1 | 4 | 4 | <0.0001 |

| | | | | | |
|---|---|---|---|---|---|
| legend: **^{a}** Denotes A/PR/8/34 immunization in mice. **^{b}** Denotes number of peptides that were up-regulated and down regulated compared to PBS immunized mice (Mock). **^{c}** Denotes total number of epitopes observed bioinformatically using GUITOPE on a protein. **^{d}** Denotes number of peptides mapped to first epitope. **^{e}** Denotes total number of mapped peptides from either up regulated and down regulated in PR8 immunized mice. **^{f}** Denotes False discovery rate of observing equivalent matching against same antigen when equal number of peptides are chosen from 10,000 random peptide library. | | | | | |

### EXAMPLE 2: Computer Architectures for Use with an Immunosignature System.

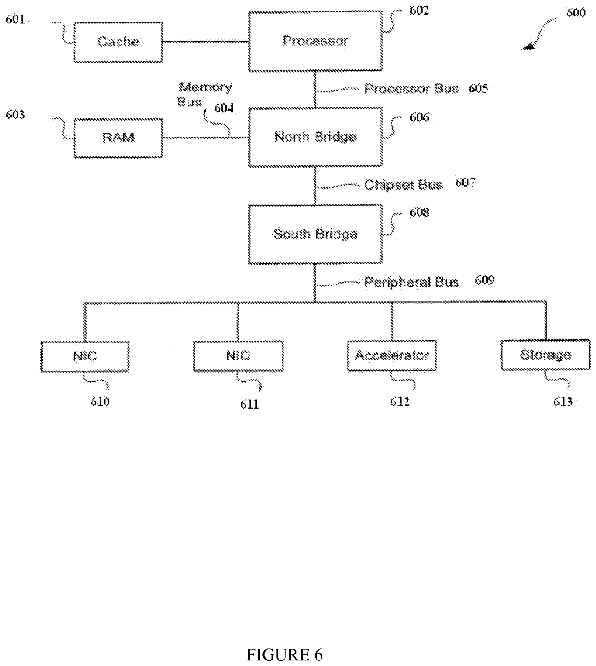

The data detected from an array of the disclosure can be analyzed by a plurality of computers, with various computer architectures. **FIGURE 6** is a block diagram illustrating a first example architecture of a computer system **600** that can be used in connection with examples of the present disclosure. As depicted in **FIGURE 6****,** the example computer system can include a processor **602** for processing instructions. Non-limiting examples of processors include: Intel Core i7TM processor, Intel Core i5TM processor, Intel Core i3TM processor, Intel XeonTM processor, AMD OpteronTM processor, Samsung 32-bit RISC ARM 1176JZ(F)-S v1.0TM processor, ARM Cortex-A8 Samsung S5PC100TM processor, ARM Cortex-A8 Apple A4TM processor, Marvell PXA 930TM processor, or a functionally-equivalent processor. Multiple threads of execution can be used for parallel processing. In some cases, multiple processors or processors with multiple cores can be used, whether in a single computer system, in a cluster, or distributed across systems over a network comprising a plurality of computers, cell phones, and/or personal data assistant devices.

### Data acquisition, processing and storage.

As illustrated in **FIGURE 6****,** a high speed cache **601** can be connected to, or incorporated in, the processor **602** to provide a high speed memory for instructions or data that have been recently, or are frequently, used by processor **602.** The processor **602** is connected to a north bridge **606** by a processor bus **605.** The north bridge **606** is connected to random access memory (RAM) **603** by a memory bus **604** and manages access to the RAM **603** by the processor **602.** The north bridge **606** is also connected to a south bridge **608** by a chipset bus **607.** The south bridge **608** is, in turn, connected to a peripheral bus **609.** The peripheral bus can be, for example, PCI, PCI-X, PCI Express, or other peripheral bus. The north bridge and south bridge are often referred to as a processor chipset and manage data transfer between the processor, RAM, and peripheral components on the peripheral bus **609.** In some architectures, the functionality of the north bridge can be incorporated into the processor instead of using a separate north bridge chip.

In some cases, system **600** can include an accelerator card **612** attached to the peripheral bus **609.** The accelerator can include field programmable gate arrays (FPGAs) or other hardware for accelerating certain processing.

### Software interface(s).

Software and data are stored in external storage **613** and can be loaded into RAM **603** and/or cache **601** for use by the processor. The system **600** includes an operating system for managing system resources; non-limiting examples of operating systems include: Linux, WindowsTM, MACOSTM, BlackBerry OSTM, iOSTM, and other functionally-equivalent operating systems, as well as application software running on top of the operating system.

In this example, system **600** also includes network interface cards (NICs) **610** and **611** connected to the peripheral bus for providing network interfaces to external storage, such as Network Attached Storage (NAS) and other computer systems that can be used for distributed parallel processing.

### Computer systems.

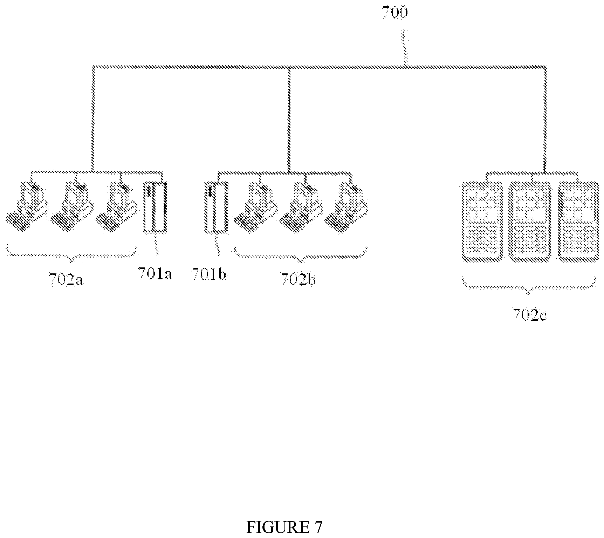

**FIGURE 7** is a diagram showing a network **700** with a plurality of computer systems **702a,** and **702b,** a plurality of cell phones and personal data assistants **702c,** and Network Attached Storage (NAS) **701a,** and **701b.** In some cases, systems **702a, 702b,** and **702c** can manage data storage and optimize data access for data stored in Network Attached Storage (NAS) **701a** and **702b.** A mathematical model can be used for the data and be evaluated using distributed parallel processing across computer systems **702a,** and **702b,** and cell phone and personal data assistant systems **702c.** Computer systems **702a,** and **702b,** and cell phone and personal data assistant systems **702c** can also provide parallel processing for adaptive data restructuring of the data stored in Network Attached Storage (NAS) **701a** and **701b.** **FIGURE 7** illustrates an example only, and a wide variety of other computer architectures and systems can be used in conjunction with the various cases of the present disclosure. For example, a blade server can be used to provide parallel processing. Processor blades can be connected through a back plane to provide parallel processing. Storage can also be connected to the back plane or as Network Attached Storage (NAS) through a separate network interface.

In some cases, processors can maintain separate memory spaces and transmit data through network interfaces, back plane, or other connectors for parallel processing by other processors. In some cases, some or all of the processors can use a shared virtual address memory space.

### Virtual systems.

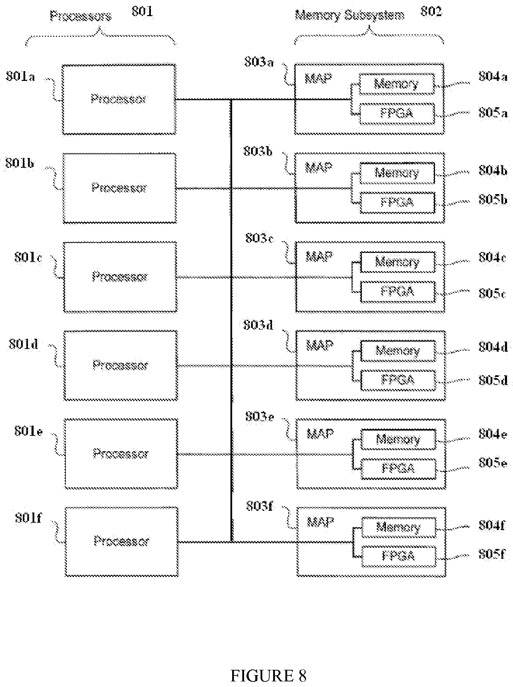

**FIGURE 8** is a block diagram of a multiprocessor computer system using a shared virtual address memory space. The system includes a plurality of processors **801a-f** that can access a shared memory subsystem **802.** The system incorporates a plurality of programmable hardware memory algorithm processors (MAPs) **803a-f** in the memory subsystem **802.** Each MAP **803a-f** can comprise a memory **804a-f** and one or more field programmable gate arrays (FPGAs) **805a-f.** The MAP provides a configurable functional unit and particular algorithms or portions of algorithms can be provided to the FPGAs **805a-f** for processing in close coordination with a respective processor. In this example, each MAP is globally accessible by all of the processors for these purposes. In one configuration, each MAP can use Direct Memory Access (DMA) to access an associated memory **804a-f,** allowing it to execute tasks independently of, and asynchronously from, the respective microprocessor **801a-f.** In this configuration, a MAP can feed results directly to another MAP for pipelining and parallel execution of algorithms.

The above computer architectures and systems are examples only, and a wide variety of other computer, cell phone, and personal data assistant architectures and systems can be used in connection with example cases, including systems using any combination of general processors, co-processors, FPGAs and other programmable logic devices, system on chips (SOCs), application specific integrated circuits (ASICs), and other processing and logic elements. Any variety of data storage media can be used in connection with example cases, including random access memory, hard drives, flash memory, tape drives, disk arrays, Network Attached Storage (NAS) and other local or distributed data storage devices and systems.

In example cases, the computer system can be implemented using software modules executing on any of the above or other computer architectures and systems. In other cases, the functions of the system can be implemented partially or completely in firmware, programmable logic devices such as field programmable gate arrays (FPGAs) as referenced in **FIGURE 8****,** system on chips (SOCs), application specific integrated circuits (ASICs), or other processing and logic elements. For example, the Set Processor and Optimizer can be implemented with hardware acceleration through the use of a hardware accelerator card, such as accelerator card **612** illustrated in **FIGURE 6****.**

### EXAMPLE 3: Immunosignaturing System.

The following example describes an automated system for Immunosignaturing.

The automated system comprises several components: 1) an automated system to receive, log, and dilute a biological sample from a subject, such as a blood or a saliva sample. The automated system contacts the biological sample with a peptide microarray of the invention.

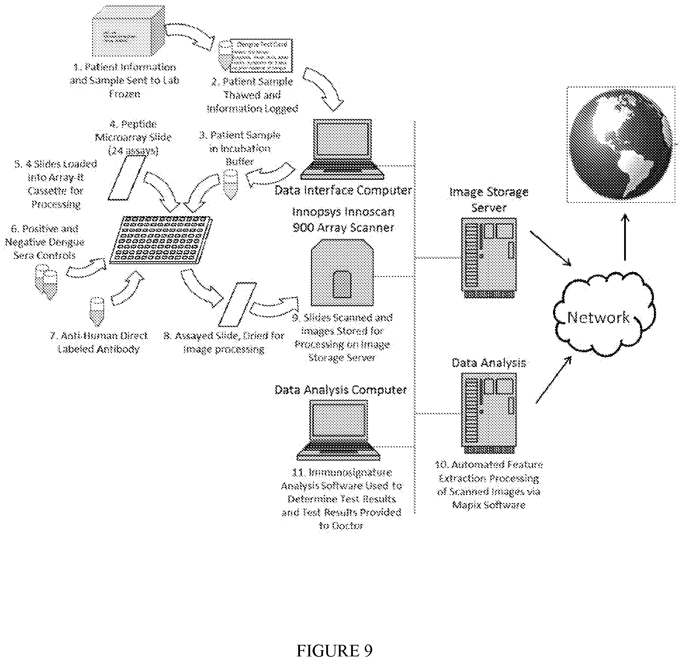

An Immunosignaturing of a subject can be obtained in an immunosignature assay of subjects consisting of the automated steps of: a) applying a diluted sample to a peptide array; b) incubating for a specific time; c) removing the sample and washing the array; d) applying a secondary antibody solution for a specific time; e) removing unbound and/or excess secondary antibody with a wash step; and f) drying and scanning the array to determine a fluorescence of an spot. **FIGURE 9** is a diagram of components of an Immunosignaturing system of the invention.

### Data collection and analysis.

Arrays are aligned and signatures determined relative to standard signatures. A standard signature can be the signature of a health subject or a reference signal of an unbound peptide.

Based on the immunosignature obtained with a system of the invention, a diagnosis can be provided. The methods of discovering therapeutic targets and deciphering Immunosignatures of the invention can be performed in any location. As illustrated in **FIGURE 9****,** the data identified with a method of discovering therapeutic targets and deciphering can be transmitted to any location. In some cases, the data in transmitted across different neighborhoods, counties, cities, states, and/or countries with, for example, a cloud networking system illustrated in

### FIGURE 9.

### EXAMPLE 4: Discovering and Deciphering Unannotated Proteins from a Pathogen.

The following example describes the validation of a method of the disclosure for the discovery of previously unidentified and/or unannotated proteins. It will be readily appreciated that the method described herein can also be applied to the characterization of insufficiently or unsatisfactorily characterized proteins.

Recently, Jagger *et al.* reported the identification of a second open reading frame in the PA-X protein, which when accessed by ribosomal frameshifting results in a novel PA-X protein with biological activity (B. W. Jagger et al., Science 337, 199 9, Jul. 13, 2012; herein "Jagger"). We show in this example the application of the methods and systems of the disclosure as a platform for antigen discovery by demonstrating that Immunosignaturing can be applied to the identification of a novel antigen of the PA-X protein.

### GuiTope Search of the PA-X Frameshift Using the Longterm Immunosignature of Murine Influenza.

Serum antibodies from mice infected with Influenza (H1N1) A/PR/8/34 were contacted with a peptide array as described in Legutki (J. B. Legutki, D. M. Magee, P. Stafford, S. A. Johnston, Vaccine 28, 4529 (May 5, 2010)), and the intensity of 283 peptides of 10,000 total peptides on a CIM10K array were correlated.

The Genbank sequence for PA from A/PR/8/34 (Accession number CY084187) was retrieved and nucleotide 598 was removed to reproduce the frameshift reported by Jagger. The resulting nucleotide sequence was translated using the online translator at www.expasy.org. The GuiTope software (R. F. Halperin, P. Stafford, J. S. Emery, K. A. Navalkar, S. A. Johnston, BMC Bioinformatics 13, 1, 2012) was configured to use the list of 156 unabsorbed peptides reported in (J. B. Legutki, D. M. Magee, P. Stafford, S. A. Johnston, Vaccine 28, 4529, May 5, 2010) as the input sequence, the CIM10K library as the background and the translated PA-X protein as the query sequence. Scores were calculated using the default substitution matrix, an inversion weight of 1, library subtracted scores, and alignment score cutoff of 10 over a moving average window of 8 amino acids.

### Construction and Probing of the Tiled PA-X Frameshift Array.

The ORF-X portion of PA-X was divided into 4 tiled peptides each with a C-terminal GSC linker to facilitate attachment to a maleimide activated aminosilane slide. Slides were activated and printed using a Nanoprint 60 with SMP2 pins. Tiled PA-X and control peptides were printed in a 21 up format spaced to fit an Array-It 24 up microarray cassette. Slides were washed to remove unconjugated peptide and blocked as previously described (J. B. Legutki, D. M. Magee, P. Stafford, S. A. Johnston, Vaccine 28, 4529, May 5, 2010; and B. A. Chase, S. A. Johnston, J. B. Legutki, Clin Vaccine Immunol 19, 352, Mar, 2012) prior to loading into the 24 up cassette. All incubations took place at room temperature on a rocking platform. Washing was done on a 96 pin microplate washer (BioTek TS405) using an optimized wash protocol. Serum applied at 1:500 with 200ul per well in 3% BSA in Phosphate Buffered Saline with 0.05% tween (PBST). Bound serum antibodies were detected with 5.0 nM anti-mouse IgG (H+L)-AlexaFluor647. Washed and dried slides were scanned in an Agilent 'C' type scanner and data extracted using GenePix. Background subtracted intensities were extracted from the GenePix results files were analyzed in Excel. Samples having a Pearson R2 greater than 0.85 were included in the final anlaysis. Significance was determined using a Student's two-tailed T test with a cutoff of 0.005.

### Identification of a Peptide Sequence which Binds the PA-X Protein.

The GuiTope software was developed to predict epitopes using the immunosignature by aligning random-sequence peptides to protein sequences (R. F. Halperin, P. Stafford, J. S. Emery, K. A. Navalkar, S. A. Johnston, BMC Bioinformatics 13, 1 (2012)). We translated the predicted PA-X transcript using the Influenza (H1N1) A/PR/8/34 genome and searched the resulting sequence with the 156 unabsorbed peptides using the GuiTope software. Two peptides aligned with library subtracted scores of 10.931 (PAMKHREPHWVIPGIIWGSC; SEQ ID NO.: 2) and 10.06 (EPMEMHDDRTMRPNGAFGSC, SEQ ID NO.: 3) which are greater than the minimum cutoff of 10 used across a moving average of 8 amino acids.

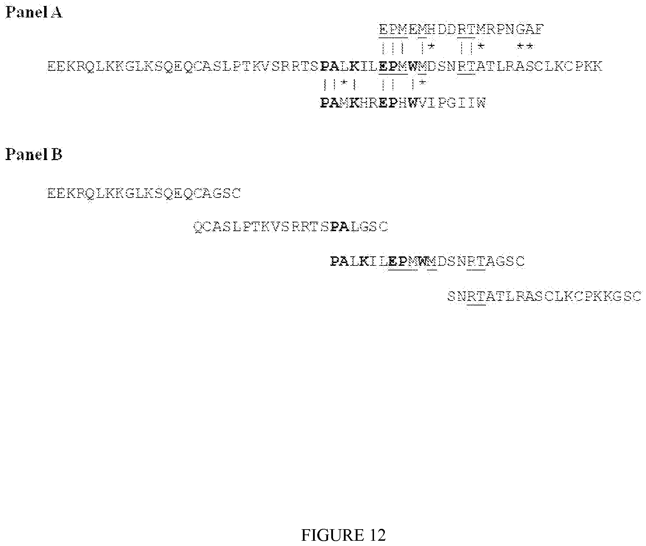

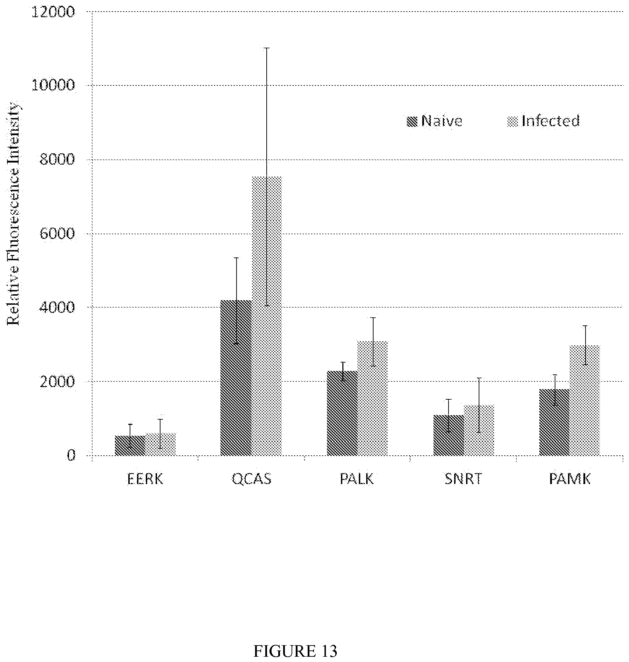

The GSC linker present on each peptide was not included in the GuiTope search to avoid artifacts. The alignment is shown in **FIGURE 12****,** Panel A with identities highlighted in bold for peptide 1 and underlined for peptide 2. To validate the prediction, four tiled peptides (**FIGURE 12****,** Panel B) representing ORF-X from A/PR/8/34 were synthesized and printed on a small microarray. The predicted epitope, PA-X3 was increasingly bound by serum antibodies in mice following infection with A/PR/8/34 (n = 12, p = 4.16x10-4) (**FIGURE 13**). Positive and negative control peptides performed as predicted.

### EXAMPLE 5: Predicting Vaccine Efficacy and Discovering Epitopes.

The following example describes an application of a method of the disclosure for the prediction of vaccine efficacy and target identifying screening.

To test the ability of Immunosignaturing to predict vaccine efficacy, a model challenge system with known individual outcomes was utilized. Five groups of twelve female BALB/c mice were immunized, individually bleed and then challenged with the Influenza H1N1 A/PR/8/34 (PR8) virus. Three groups had been previously vaccinated with inactivated viruses. These were formalin inactivated PR8 (killed PR8), with the commercially available 2006/2007 and 2007/2008 seasonal trivalent influenza vaccines. The two seasonal influenza vaccines share the same A/Wisconsin/67/2005 (H3N2) and B/Malaysia/2506/2004, but vary in the H1N1 portion containing A/New Caledonia/20/99 and A/Solomon Islands/3/2006 respectively. In addition to a mock vaccination group, a fifth group was vaccinated a single time with a sublethal dose of the live PR8 virus.

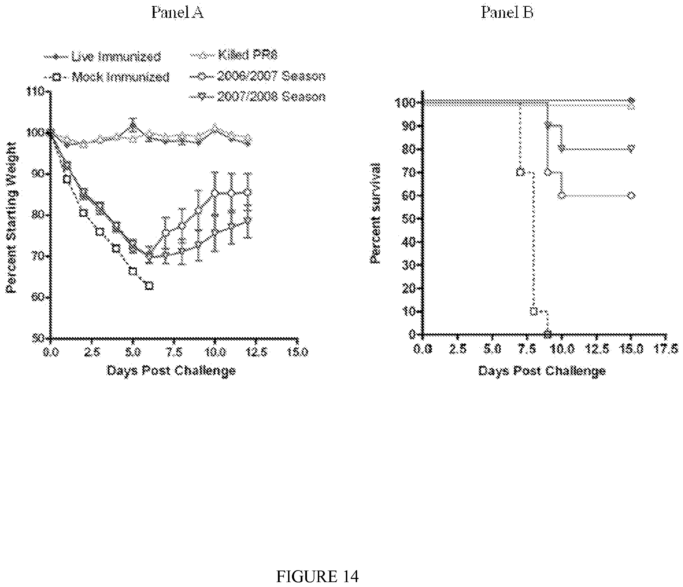

The mice were challenged with 2-5 MLDs of active PR8 and results are presented in **FIGURE 14. FIGURE 14** illustrates the outcomes of immunized mice following lethal challenge with Influenza A/PR/8/34. Mice were immunized with PBS (mock), a live sublethal dose of A/PR/8/34, inactivated A/PR/8/34, the 2006/2007 seasonal influenza vaccine or the 2008/2007 seasonal influenza vaccine. Mice were challenged intranasally with 5 x 10⁵ PFU per mouse. The average daily percent starting weight is graphed in Panel A where the average is calculated based on the surviving mice and error bars represent the standard deviation. Survival Curves are presented in Panel B and represent the percentage of mice surviving following challenge.

No mice immunized with sublethal challenge or killed PR8 succumbed to the challenge and none of these mice had a symptomatic infection as evidenced by the absence of fur ruffling and weight loss. The two seasonal vaccines were partially protective resulting in 60% and 80% survival. All surviving mice had significant weight loss. Sera was collected two days prior to challenge and used to probe the CIM10K array and to establish an Immunosignature.

### A Systems Vaccine Baseline Comparison.

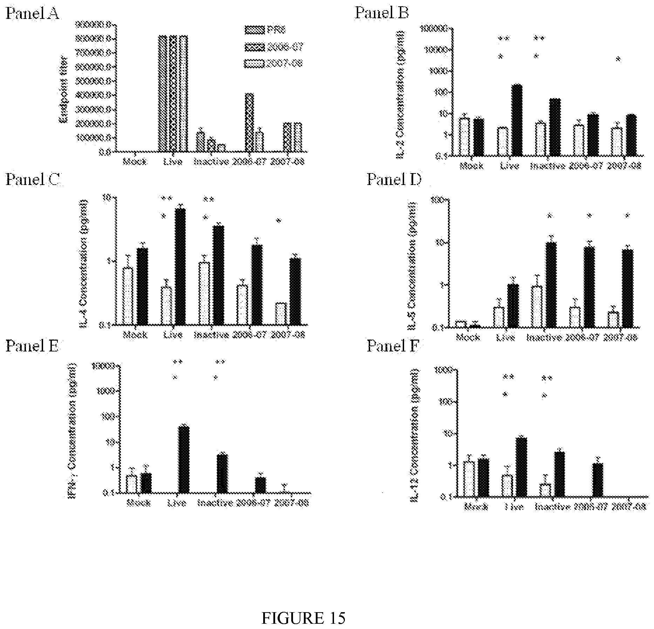

In order to create a baseline for comparison to Immunosignaturing, the antibody and T-cell responses in the vaccine groups were assessed. Serum antibodies against the viruses were assessed by ELISA two days before challenge (**FIGURE 15**). **FIGURE 15** illustrates traditional measures of the immune response. Prior to challenge serum was collected from all mice. The amount of antigen specific circulating IgG was measured for inactive PR8, the 2006-2007 and 2007-2008 seasonal vaccines by endpoint titer and is graphed in Panel A. Error bars are the standard deviation of triplicate measurements of pooled sera. Two mice per group were euthanized and individual splenocytes stimulated with inactivated virus and cytokines secreted 48 hours measured using a Bioplex Th1/Th2 cytokine assay and graphed in (Panels B through F). Values plotted represent the mean +/- standard deviation for four measurements. Significance is indicated as * significant vs media and ** significant vs mock immunized. As evident in Panel A, only the mice receiving the live vaccine or the killed PR8 vaccine had detectable antibodies against PR8. The two seasonal vaccines, which were only partially protective against the PR8 challenge did not have a detectable response to PR8.

In order to assess T-cell responses, on the day of challenge, two mice per group were sacrificed and spleens harvested. For the splenocyte stimulation assay, 2.5 x 10⁵ cells were seeded per well in the presence of inactivated whole virus. After 48 hours, supernatants were harvested and cytokine secretion was assayed using the BioRad Luminex Th1/Th2 assay kit. Concentrations of secreted cytokines are presented in **FIGURE 15****,** Panels A-F. The mice receiving the live vaccine stimulated a strong T cell response, secreting all cytokines assayed including those associated with both Th1 and Th2 helper cells. Of the inactive vaccines, only the killed PR8 recipients secreted significant levels of all cytokines when stimulated by inactive virus. The seasonal vaccines did secrete IL-4 and IL-5 indicating it was primarily a CD4-Th2 response. These cytokine profiles demonstrate that the immune response produced by the killed PR8 strain was Th2 biased. This suggests an immune environment favorable to the development of the humoral arm. It was not technically possible to establish a T-cell profiles for all mice in the study.

### Live and Inactive Influenza Immunizations Produce Different Immunosignatures.

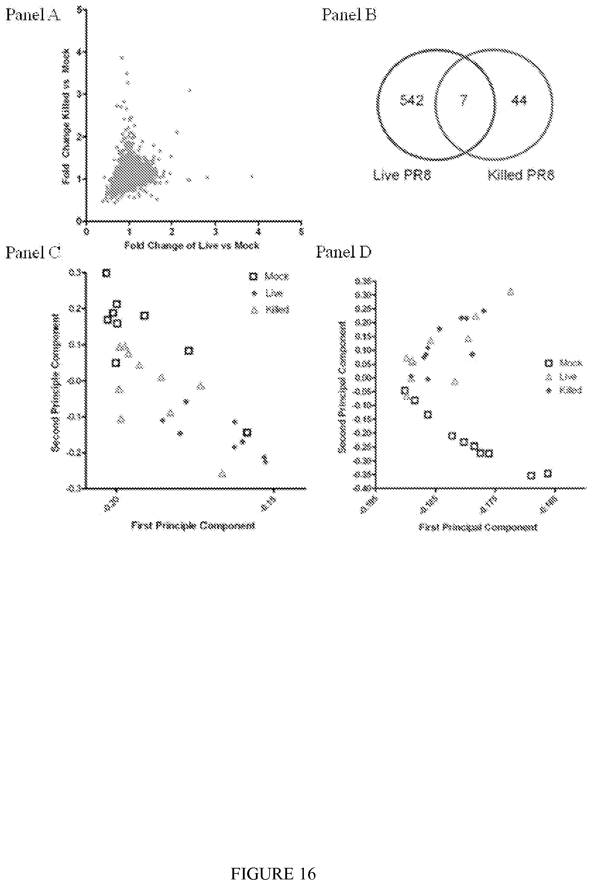

The live and killed PR8 vaccines were equally protective against challenge. The ELISA against whole virus in **FIGURE 15****,** Panel A demonstrated that the live and inactivated influenza vaccines produce different intensities of antibody response. The differences in peptides recognized by each vaccine group over naives are seen in a scatterplot in **FIGURE 16****,** Panel A. Using selection criteria of FDR-corrected p < 0.05 and fold-change >1.3-fold, serum from live influenza recognizes 10.75 x the number of peptides as the inactive vaccine serum.

The two vaccines have 7 peptides recognized in common, one would expect less than one peptide recognized by chance between similarly sized lists (**FIGURE 16****,** Panel B). A Principal Components Analysis (PCA) plot displays the relative difference among and between groups using variance as the X and Y scalar values. All 593 peptides recognized by either group of mice clearly separate the live from inactive immunized animals (**FIGURE 16****,** Panel C). A Support Vector Machine (SVM) shows 0% leave-one-out cross-validation (LOOCV) error when asked to predict the classes. Analysis of the overlapping peptides shows the live- and inactive-vaccinated mice cluster together, and are separate from the mock immunized (**FIGURE 16****,** Panel D). The larger number of peptides in the live vaccine Immunosignature can be due to the dose amplifying effect of viral replication or additional epitopes not present in the inactivated, and presumably disassembled viron. Taken together these data demonstrate that the two protective vaccines have quite distinct Immunosignatures.

### Immunosignaturing can Distinguish Closely Related Vaccines.

To evaluate the capacity of Immunosignaturing for fine scale profiling, random peptide arrays with serum from mice immunized with the inactivated seasonal influenza vaccines were probed. Two versions of the 10K array were used: pooled samples were tested on the CIM10K version 1 (CIM10Kv1) and individual samples were tested the CIM10K version 3 (CIM10Kv3). CIM10Kv3 incorporates numerous technical improvements over CIM10K, and it was chosen to evaluate the individual mice.

**FIGURE 16** demonstrates that Immunosignature can distinguish weaker inactive vaccines from a more potent one. The Immunosignature on the CIM10Kv3.0 was compared between the two seasonal vaccines and the killed PR8 vaccine first in an ANOVA where 55 peptides at a p < 0.0005 (5 false positives) were capable of separating the three vaccines. Variance amongst individuals is represented in a plot of the first and second principal components in (**FIGURE 16****,** Panel A). The first comparison asked for peptides different from the grand mean across the three vaccines using one-way ANOVA at p < 0.0005. This comparison yielded 55 peptides capable of separating the three vaccines with 0% LOOCV error in an SVM (**FIGURE 16****,** Panel A).

The second comparison compared each vaccine separately against the mock-immunized mice using the Student's T-test. The number of peptides significantly changed compared to mock were different between vaccines. Overlap between the mock compared peptides is shown in the Venn Diagram in **FIGURE 16****,** Panel B. Comparisons between vaccinated and naive mice were made using pooled sera on the CIM10Kv1.0 using a minimum 1.3 fold increase in normalized fluorescence units in sera from immunized over mocks and a p-value of less than 0.05 using the Benjamini and Hochberg multiple test correction. Overlap between these lists is shown in the Venn Diagram in (**FIGURE 16****,** Panel B).

More overlap is seen between the two seasonal vaccines than with the killed PR8. The two seasonal vaccine formulations differ in the H1N1 strain included. This pattern is consistent on both the CIM10Kv1 and CIM10Kv3 microarrays. This demonstrates that Immunosignatures are sensitive enough to detect subtle differences in vaccine compositions.

### The Immunosignature of a Known Protective Response can Predict Outcome Following Challenge.

Herein we demonstrate that the Immunosignature of a known protective response can predict vaccine efficacy. The SAM algorithm uses a per-mutated T-test and was used to select 25 peptides capable of distinguishing live from mock immunized as the training set with a false positive rate of one peptide (1/25, or FP=4%). These 25 peptides included the overlap peptides between the live and killed PR8 Immunosignatures. To overcome the influences of varying affinities for peptides, we utilized a binary classifier that bins array features based on whether a certain cutoff score has been reached. These binary scores were used to calculate the group average of pairwise Hamming distances as the number of binary differences between Immunosignatures shown in **TABLE 7**.

**TABLE 7**

| **TABLE 7** | | |
|---|---|---|
| | **Mock** | **Live PR8** |
| Mock | 3.4 ±1.2 | 21.0 ± 3.0 |
| Live | 21.0 ± 3.2 | 4.5 ± 4.4 |
| Killed PR8 | 8.3 ± 4.0^{f} | 16.4 ± 4.1^{e} |
| 2006/2007 | 5.9 ± 4.7 | 18.7 ± 5.0 |
| 2007/2008 | 5.6 ± 1.6 | 19.0 ± 3.1 |

| | | |
|---|---|---|
| Legend: ^{a} The log2 of ratios between individuals were calculated for each of the peptides capable of distinguishing live from mock immunized mice using SAM; ^{b} Ratios were binned as 1 or 0 based on a cutoff of the peptides 10^{th} percentile in the live immunized mice; ^{c} The Hamming Distance was calculated using the binary scores; ^{d} Average Hamming Distance ± standard deviation. ^{e} Statistically distinct from the seasonal and live vaccines with a two tailed T test p = 6.5 x 10⁻⁵; ^{f} Statistically distinct from the seasonal and mock vaccines with a two tailed T test p = 1.39 x 10⁻⁶. | | |

Seasonal vaccines were used as the test set on the same 25 peptides. The mice immunized with killed PR8, were found to be closer to the live immunized mice and farther from the mock immunized than those receiving the seasonal vaccines. This fits with the inactive PR8 vaccine being the one that imparted complete symptom free protection, while the seasonal vaccines only afforded partial protection. Immunosignature based prediction of the killed PR8 as the most protective vaccine reflects the relative ELISA titers and the cytokine expression patterns. Had the immunosignature been the only assay used, it would have picked the correct vaccine. The data demonstrates the ability of the immunosignature to aid in vaccine development by selecting the vaccine with the highest protective efficacy.

### Seasonal Vaccine Recipients have Distinct Immunosignatures which Correlate with Outcome Following PR8 Challenge.

Mice immunized with the seasonal vaccines were partially protected against challenge with the PR8 strain. None of the splenocytes from seasonal vaccine recipients secreted IL-12 or IFN-γ suggesting that partial protection was conveyed in the absence of a strong CD8 response.

**FIGURE 18** demonstrates that Immunosignature can predict that antibody crossreactivity to NA195-219 was important in protecting seasonal vaccine recipients from the PR8. The whole virus ELISA endpoint titers for the 2006-2007 vaccine recipients that survived or died following PR8 challenge are shown in (**FIGURE 18****,** Panel A) where the horizontal line represents the group mean and each point represents an individual mouse. (**FIGURE 18****,** Panel A) does not indicate an explanatory trend within the groups.

The Immunosignature was compared between the seasonal vaccine recipients for both years that survived or succumbed to challenge. Using a two tailed T-test with a cutoff of p<0.005, 94 peptides were identified as significantly different and were capable of a 100% LOOCV accuracy using SVM. In a PCA plot, the surviving mice grouped with the mice immunized with killed PR8 (**FIGURE 18****,** Panel B). The variance among all individuals receiving an inactive vaccine is presented in (**FIGURE 18****,** Panel B) as a plot of the first and second principal components.

The 38 peptides which were at least 1.3 fold less recognized by those that succumbed were used to predict the epitope in neuraminidase (**FIGURE 18****,** Panel C) where the GUItope score is on the y-axis and amino acid on the x-axis. Antibody reactivity from pooled sera to the strongest predicted epitope are plotted in (**FIGURE 18****,** Panel D) as the mean +/- standard deviation of replicate arrays. The PR8 immunized mice were not included in the selection of these 94 peptides. This suggests that immunosignatures could be used to predict the individual outcome for vaccinees upon infection.

### The Immunosignature can be Bioinformatically Tracked back to the Epitopes on the A/PR/8/34 Proteins.

The experiments described above indicate that Immunosignatures can produce surrogates of protection. It would be useful if these surrogates could also indicate correlates of protection, for example the actual protective epitopes in the virus.

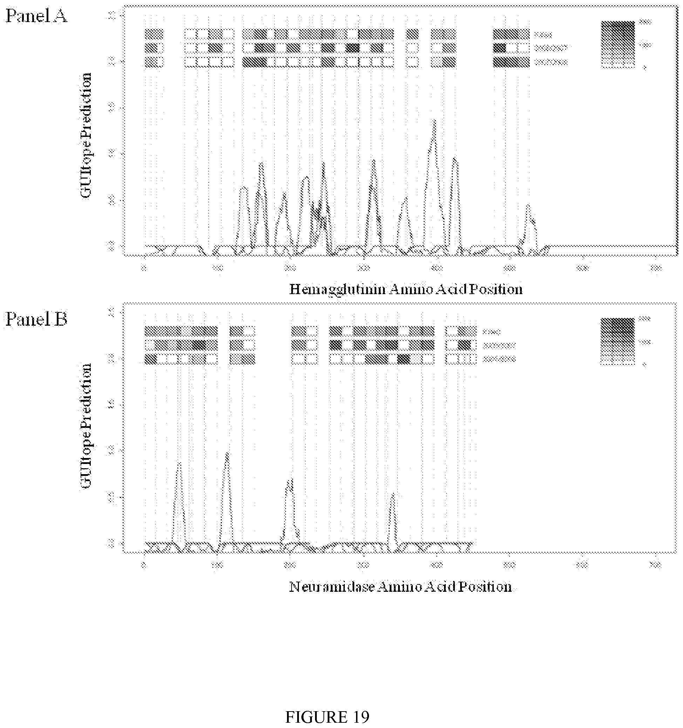

To assess the breadth of epitopes recognized by the inactive vaccines, we reduced our selection of peptides to those that had increased signals following immunization. Peptides binding antibodies raised by immunization with killed PR8 were searched against the PR8 protein sequences and the results for HA and NA plotted in **FIGURE 19. FIGURE 19** illustrates an Immunosignature on random peptide arrays can be interpreted to determine epitopes recognized by each vaccine. The peptides determined to be increasingly recognized in vaccinated over naive mice were used to GUItope search the protein sequences for the PR8 hemagglutinin (**FIGURE 19****,** Panel A) and neuraminase (**FIGURE 19****,** Panel B). Peptides were selected by comparing vaccinated and naive mice using pooled sera on the CIM10Kv1.0 using a minimum 1.3 fold increase in immunized over mocks and an FDR adjusted p value of less than 0.05. For both proteins, the line graph represents the GUItope prediction score for each amino acid. Recognition of actual epitope sequences is presented in the blocks above each line graph. Blocks represent the amino acids (x-axis) covered by tiled peptides containing epitope sequence. Block colors represent the binding intensity of antibodies from pooled sera from each vaccine to those peptides. The color scale used is in the upper right of each panel.

Due to the close homology between influenza strains, common epitopes were predicted yet the killed PR8 vaccinated mice recognized unique sequences. To test these predictions, the pooled sera of the immunized mice were used to probe a tiled peptide array containing most of the PR8 HA and NA sequences. From the sequences present on the tiled peptide array, all of the predictions were supported but not all epitopes were predicted. Exclusion of these epitopes from the predicted list can be due to the stringent false discovery corrections used to select the peptides. If the false discovery correction is removed, the percentage of epitopes in agreement between the GuiTope prediction and the epitope array increases. Interestingly only the mice immunized with killed PR8 were predicted to bind epitopes on NA, including 203-SWRKKILRTQES-209 (SWRKKILRTQES: SEQ ID NO. 4) whose homolog in the 2009 A H1N1 virus is a known neutralizing epitope. Not all peptides included in the immunosignature were predicted by GUItope to align to actual sequences. These peptides can be recognized by antibodies to conformational epitopes.

These results demonstrate that immunosignaturing can accurately detect antibodies raised elicited endogenously against linear epitopes of biological immunogens.

### EXAMPLE 6: Discovering and Deciphering Peptides Capable of Eliciting an Antigenic Response.

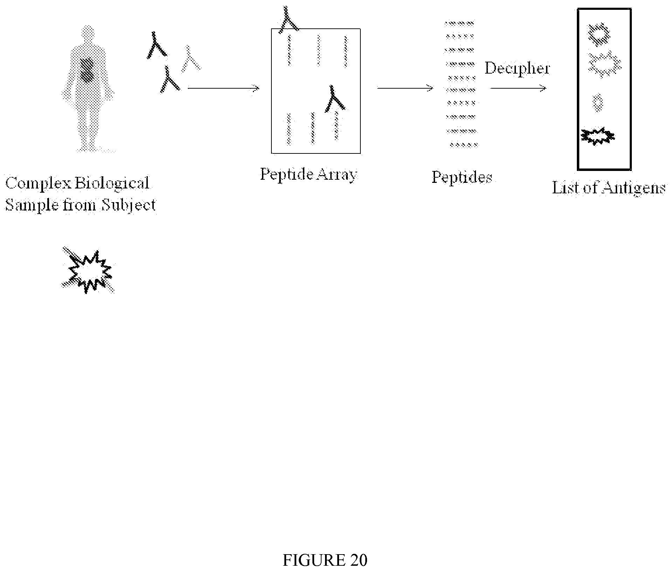

**FIGURE 20** is an overall schematic of one application of the invention in target identifying screening.

A complex biological sample, ex: sera, from an individual suffering from a disorder of interest is contacted with a peptide array; a binding of antibodies in the sera to peptides on the array to generate a disease immune profile is detected; the disease immune profile of the individual is compared to a normal control and differentially bound peptides are identified based on one or both of: (i) peptides that bound more antibody in the disease immune profile compared to normal control; and (ii) peptides that bound less antibody in the disease immune profile compared to normal control; wherein proteins corresponding to the differentially bound peptides are therapeutic targets for the disease of interest.

One of skill in the art will appreciate that a plurality of the arrays described herein can be used with such a method. One of skill in the art will also appreciate that a plurality of methods of detection can be used in conjunction with such methods. One of skill in the art will also appreciate that a plurality of peptides capable of eliciting an antigenic response to a plurality of different conditions can be discovered and deciphered with the methods described herein. In some cases, proteins corresponding to the differentially bound peptides are therapeutic targets for the disease of interest.

## Claims

1. A method of screening for therapeutic targets for an autoimmune disorder comprising:
a) contacting a first peptide array with a first biological sample from an individual with an autoimmune disorder;
b) detecting binding of at least one antibody in the first biological sample with the first peptide array to obtain a first immunosignature profile;
c) contacting a second peptide array with a second biological sample, consisting of a control sample derived from an individual without the autoimmune disorder;
d) detecting binding of at least one antibody in the control sample with the second peptide array to obtain a second immunosignature profile;
e) comparing the first immunosignature profile to the second immunosignature profile and identifying peptide(s) that are associated with the at least one antibody downregulated in the first biological immunosignature profile; and
f) identifying proteins that correspond to the identified peptides as therapeutic targets for the autoimmune disorder;
wherein the autoimmune disorder is selected from the group consisting of Type 1 diabetes, rheumatoid arthritis, multiple sclerosis, inflammatory bowel disease, systemic lupus erythematosus, psoriasis and scleroderma.

2. The method of screening for therapeutic targets of claim 1, wherein the protein(s) are unannotated.

3. The method of screening for therapeutic targets of claim 1, further comprising contacting the first peptide array, the second peptide array, or both with a competitive inhibitor.

4. The method of screening for therapeutic targets of any of claims 1-3, wherein the protein(s) have a frameshift.

5. The method of screening for therapeutic targets of any of claims 1-4, wherein the therapeutic targets are an epitope of the protein.

6. The method of screening for therapeutic targets of any of claims 1-5, wherein the first peptide array, or the second peptide array, or both comprise between 500-1,000,000 peptides.

7. The method of screening for therapeutic targets of any of claims 1-6, wherein the peptides on the first peptide array, the second peptide array, or both are between 8 and 35 residues in length.

8. The method of screening for therapeutic targets of any of claims 1-7, wherein the peptides on the first peptide array, the second peptide array, or both have an average spacing ranging from 2-4 nm.

9. The method of screening for therapeutic targets of any of claims 1-8, wherein no more than about 5 nl to about 1 µl of the first biological sample is contacted with the first peptide array.

10. The method of screening for therapeutic targets of any of claims 1-8 or 9, wherein the therapeutic targets comprise a vaccine target.

11. The method of screening for therapeutic targets of any of claims 1-8 or 9, where the therapeutic targets suppress antibody secretion by a B-cell.

12. The method of screening for therapeutic targets of any of claims 1-8 or 9-11, wherein the autoimmune disorder is Type I diabetes.

## Patentansprüche

1. Ein Verfahren zum Screening therapeutischer Zielmoleküle für eine Autoimmunerkrankung, umfassend:
a) Kontaktieren einer ersten Peptidanordnung mit einer ersten biologischen Probe von einem Individuum mit einer Autoimmunerkrankung;
b) Erfassen einer Bindung von mindestens einem Antikörper in der ersten biologischen Probe mit der ersten Peptidanordnung, um ein erstes Immunsignatur-Profil zu erhalten;
c) Kontaktieren einer zweiten Peptidanordnung mit einer zweiten biologischen Probe, bestehend aus einer Kontrollprobe von einem Individuum ohne Autoimmunerkrankung;
d) Erfassen einer Bindung von mindestens einem Antikörper in der Kontrollprobe mit der zweiten Peptidanordnung, um ein zweites Immunsignatur-Profil zu erhalten;
e) Vergleichen des ersten Immunsignatur-Profils mit dem zweiten Immunsignatur-Profil und Identifizieren des Peptids/der Peptide, das/die mit dem mindestens einen Antikörper verbunden ist/sind, heruntergeregelt in dem ersten biologischen Immunsignatur-Profil; und
f) Identifizieren von Proteinen, die den identifizierten Peptiden als therapeutische Zielmoleküle für die Autoimmunerkrankung entsprechen;
wobei die Autoimmunerkrankung ausgewählt ist aus der Gruppe bestehend aus Typ 1-Diabetes, rheumatoider Arthritis, multipler Sklerose, chronisch-entzündlicher Darmerkrankung, systemischem Lupus erythematodes, Psoriase und Sklerodermie.

2. Das Verfahren zum Screening therapeutischer Zielmoleküle nach Anspruch 1, wobei das/die Protein(e) nicht annotiert sind.

3. Das Verfahren zum Screening therapeutischer Zielmoleküle nach Anspruch 1, ferner umfassend das Kontaktieren der ersten Peptidanordnung, der zweiten Peptidanordnung oder beiden mit einem kompetitiven Inhibitor.

4. Das Verfahren zum Screening therapeutischer Zielmoleküle nach einem der Ansprüche 1 - 3, wobei das/die Protein(e) eine Rastermutation haben.

5. Das Verfahren zum Screening therapeutischer Zielmoleküle nach einem der Ansprüche 1 - 4, wobei die therapeutischen Zielmoleküle ein Epitop des Proteins sind.

6. Das Verfahren zum Screening therapeutischer Zielmoleküle nach einem der Ansprüche 1 - 5, wobei die erste Peptidanordnung oder die zweite Peptidanordnung oder beide zwischen 500-1.000.000 Peptiden umfassen.

7. Das Verfahren zum Screening therapeutischer Zielmoleküle nach einem der Ansprüche 1 - 6, wobei die Peptide der ersten Peptidanordnung, der zweiten Peptidanordnung oder beiden zwischen 8 und 35 Resten in der Länge sind.

8. Das Verfahren zum Screening therapeutischer Zielmoleküle nach einem der Ansprüche 1 - 7, wobei die Peptide der ersten Peptidanordnung oder der zweiten Peptidanordnung oder beiden einen durchschnittlichen Zwischenraum von 2-4 nm haben.

9. Das Verfahren zum Screening therapeutischer Zielmoleküle nach einem der Ansprüche 1 - 8, wobei nicht mehr als ungefähr 5 µl bis ungefähr 1 µl der ersten biologischen Probe mit der ersten Peptidanordnung in Kontakt kommen.

10. Das Verfahren zum Screening therapeutischer Zielmoleküle nach einem der Ansprüche 1 - 8 oder 9, wobei die therapeutischen Zielmoleküle ein Impfstoff-Zielmolekül umfassen.

11. Das Verfahren zum Screening therapeutischer Zielmoleküle nach einem der Ansprüche 1 - 8 oder 9, wobei die therapeutischen Zielmoleküle eine Antikörper-Sekretion durch eine B-Zelle unterbinden.

12. Das Verfahren zum Screening therapeutischer Zielmoleküle nach einem der Ansprüche 1 - 8 oder 9 - 11, wobei die Autoimmunerkrankung Typ 1-Diabetes ist.

## Revendications

1. Procédé de dépistage destiné à des cibles thérapeutiques destinées à une maladie auto-immune comprenant :
a) la mise en contact d'un premier réseau de peptides avec un premier échantillon biologique provenant d'une personne comportant une maladie auto-immune ;
b) la détection de la liaison d'au moins un anticorps dans le premier échantillon biologique avec le premier réseau de peptides pour obtenir un premier profil d'immunosignature ;
c) la mise en contact d'un second réseau de peptides avec un second échantillon biologique, comprenant un échantillon de contrôle dérivé à partir d'un individu sans la maladie auto-immune ;
d) la détection de la liaison d'au moins un anticorps dans l'échantillon de contrôle avec le second réseau de peptides pour obtenir un second profil d'immunosignature ;
e) la comparaison du premier profil d'immunosignature au second profil d'immunosignature et l'identification du ou des peptides qui sont associés audit moins un anticorps régulé négativement dans le premier profil d'immunosignature biologique ; et
f) l'identification des protéines qui correspondent aux peptides identifiés comme cibles thérapeutiques pour la maladie auto-immune ;
dans lequel la maladie auto-immune est sélectionnée dans le groupe constitué par le diabète de type 1, la polyarthrite rhumatoïde, la sclérose en plaques, la maladie abdominale inflammatoire de l'intestin, le lupus érythémateux systémique, le psoriasis et la sclérodermie.

2. Procédé de dépistage destiné à des cibles thérapeutiques selon la revendication 1, dans lequel la ou les protéines sont sans annotation.

3. Procédé de dépistage destiné à des cibles thérapeutiques selon la revendication 1, comprenant en outre la mise en contact du premier réseau de peptides, du second réseau de peptides, ou les deux à la fois avec un inhibiteur compétitif.

4. Procédé de dépistage destiné à des cibles thérapeutiques selon l'une quelconque des revendications 1 à 3, dans lequel la ou les protéines présentent un déphasage.

5. Procédé de dépistage destiné à des cibles thérapeutiques selon l'une quelconque des revendications 1 à 4, dans laquelle les cibles thérapeutiques sont un épitope de la protéine.

6. Procédé de dépistage destiné à des cibles thérapeutiques selon l'une quelconque des revendications 1 à 5, dans lequel le premier réseau de peptides, ou le second réseau de peptides, ou les deux comprennent entre 500 et 1 000 000 de peptides.

7. Procédé de dépistage destiné à des cibles thérapeutiques selon l'une quelconque des revendications 1 à 6, dans laquelle les peptides sur le premier réseau de peptides, le second réseau de peptides, ou les deux ont entre 8 et 35 résidus de longueur.

8. Procédé de dépistage destiné à des cibles thérapeutiques selon l'une quelconque des revendications 1 à 7, dans lequel les peptides sur le premier réseau de peptides, le second réseau de peptides, ou les deux présentent un espacement moyen allant de 2 à 4 nm.

9. Procédé de dépistage destiné à des cibles thérapeutiques selon l'une quelconque des revendications 1 à 8, dans lequel pas plus d'environ 5 µl à environ 1 µl du premier échantillon biologique est mis en contact avec le premier réseau de peptides.

10. Procédé de dépistage destiné à des cibles thérapeutiques selon l'une quelconque des revendications 1 à 8 ou 9, dans lequel les cibles thérapeutiques comprennent un vaccin cible.

11. Procédé de dépistage destiné à des cibles thérapeutiques selon l'une quelconque des revendications 1 à 8 ou 9, dans lequel les cibles thérapeutiques suppriment la sécrétion d'anticorps par une cellule B.

12. Procédé de dépistage destiné à des cibles thérapeutiques selon l'une quelconque des revendications 1 à 8 ou 9 à 11, dans lequel la maladie auto-immune est le diabète de type 1.
